(19) Europäisches Patentamt
European Patent Office
Office européen des brevets

(11) Veröffentlichungsnummer: **0 084 775**
**A1**

(12) # EUROPÄISCHE PATENTANMELDUNG

(21) Anmeldenummer: **83100045.0**

(22) Anmeldetag: **05.01.83**

(51) Int. Cl.³: **C 07 C 149/26,** C 07 D 307/935, C 07 C 177/00, C 07 D 333/18, C 07 D 213/32, A 61 K 31/19, A 61 K 31/215, A 61 K 31/557

(30) Priorität: **21.01.82 DE 3201764**

(43) Veröffentlichungstag der Anmeldung: **03.08.83 Patentblatt 83/31**

(84) Benannte Vertragsstaaten: **AT BE CH DE FR GB IT LI NL SE**

(71) Anmelder: **Merck Patent Gesellschaft mit beschränkter Haftung, Frankfurter Strasse 250, D-6100 Darmstadt (DE)**

(72) Erfinder: **Riefling, Bernhard, Dr., Zöllerstrasse 28, D-6100 Darmstadt-Arheilgen (DE)**
Erfinder: **Radunz, Hans-Eckart, Dr., Am Klingenteich 5, D-6109 Mühltal-4 (DE)**
Erfinder: **Minck, Klaus-Otto, Dr., Büchestrasse 8, D-6105 Ober-Ramstadt (DE)**
Erfinder: **Schliep, Hans-Jochen, Dr., Weingartenstrasse 16, D-6109 Mühltal (DE)**
Erfinder: **Melzer, Guido, Dr., Mörikestrasse 6, D-6238 Hofheim/Taunus (DE)**

(54) **13-Thia-Prostacycline.**

(57) 13-Thia-Prostacycline der Formel I

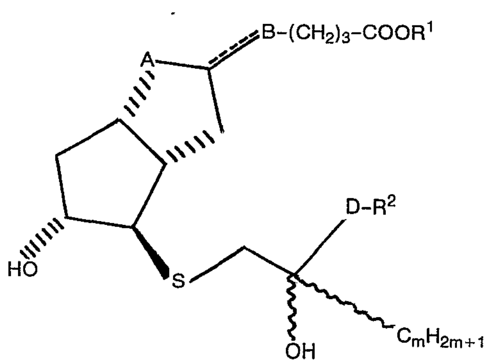

worin
A -O-, $-CH_2-$ oder eine Bindung,
B $-CH_2-$ oder =CH-,
D eine Bindung, Alkylen mit 1–3 C-Atomen, cis-Alkenylen mit 2–5 C-Atomen oder Alkinylen mit 2–5 C-Atomen,
m 0, 1, 2 oder 3,
$R^1$ H, Alkyl mit 1–4 C-Atomen, Aryl mit 6–12 C-Atomen oder $-C_6H_4-NH-CO-R^3$,
$R^2$ Alkyl mit 1–7 C-Atomen, durch Halogen substituiertes Alkyl mit 1–7 C-Atomen, Cycloalkyl mit 5–6 C-Atomen, durch Alkyl mit 1–4 C-Atomen substituiertes Cycloalkyl mit 5–6 C-Atomen, Phenyl, durch F, Cl, Br, Alkyl mit 1–4 C-Atomen, OH, $OCH_3$ oder $CF_3$ substituiertes Phenyl, Pyridyl, Naphthyl, Thienyl, oder, falls D Alkylen mit 1–3 C-Atomen bedeutet, auch Alkoxy mit 1–4 C-Atomen, Alkylthio mit 1–4 C-Atomen, Phenoxy oder durch F, Cl, Br, Alkyl mit 1–4 C-Atomen, OH, $OCH_3$ oder $CF_3$ substituiertes Phenoxy,
$R^3$ $NH_2$, $CH_3$ oder Phenyl
bedeuten,
sowie, falls $R^1$ = H ist, deren physiologisch unbedenkliche Metall- und Ammoniumsalze besitzen bei guter Verträglichkeit wertvolle pharmakologische Eigenschaften. Sie zeigen Wirkungen auf den Kreislauf, vor allem blutdrucksenkende Wirkungen sowie positiv inotrope Wirkungen auf die Herzkraft, aber auch thrombozyten-aggregationshemmende Wirkungen.

EP 0 084 775 A1

# 13-Thia-Prostacycline

Die Erfindung betrifft 13-Thia-prostacycline der allgemeinen Formel I

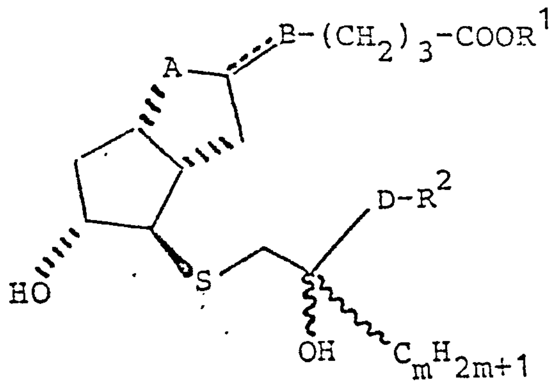

(I)

worin

A -O-, $-CH_2-$ oder eine Bindung,

B $-CH_2-$ oder =CH-,

D eine Bindung, Alkylen mit 1 - 3 C-Atomen, cis-Alkenylen mit 2 - 5 C-Atomen oder Alkinylen mit 2 - 5 C-Atomen,

m 0, 1, 2 oder 3,

$R^1$ H, Alkyl mit 1 - 4 C-Atomen, Aryl mit 6 - 12 C-Atomen oder $-C_6H_4-NH-CO-R^3$,

$R^2$ Alkyl mit 1 - 7 C-Atomen, durch Halogen substituiertes Alkyl mit 1 - 7 C-Atomen, Cycloalkyl mit 5 - 6 C-Atomen, durch Alkyl mit 1 - 4 C-Atomen substituiertes Cycloalkyl mit 5 - 6 C-Atomen, Phenyl, durch F, Cl, Br, Alkyl mit 1 - 4 C-Atomen, OH, $OCH_3$ oder $CF_3$ substituiertes Phenyl, Pyridyl, Naphthyl, Thienyl,

oder, falls D Alkylen mit 1 - 3 C-Atomen bedeutet, auch Alkoxy mit 1 - 4 C-Atomen, Alkylthio mit 1 - 4 C-Atomen, Phenoxy oder durch F, Cl, Br, Alkyl mit 1 - 4 C-Atomen, OH, $OCH_3$ oder $CF_3$ substituiertes Phenoxy,

$R^3$ $NH_2$, $CH_3$ oder Phenyl

bedeuten und

 anzeigt, daß diese Bindungen α- oder ß-ständig sein können, wobei eine der Bindungen α- und die andere ß-ständig ist,

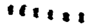 anzeigt, daß diese Bindung α-ständig ist,

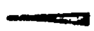 anzeigt, daß diese Bindung ß-ständig ist,

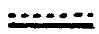 anzeigt, daß diese Bindung je nach Bedeutung von B ein Einfach- oder Doppelbindung ist, wobei die Einfachbindung α- oder ß-ständig und die Doppelbindung in der E- oder Z-Konfiguration sein kann,

sowie, falls $R^1$ = H ist, deren physiologisch unbedenkliche Metall- und Ammoniumsalze.

Seit einiger Zeit haben Verbindungen pharmakologisches und medizinisches Interesse gewonnen, welche unter dem Begriff "Prostacycline" zusammengefaßt werden können. Prostacyclin oder $PGI_2$, ein kürzlich isolierter Naturstoff aus der Familie der Prostaglandine, zeichnet sich durch ausgeprägte thrombozytenaggregationshemmende Eigenschaften aus (The Lancet 1977, 18). Die physiologischen Wirkungen von Prostaglandinen und Prostacyclinen sind jedoch sowohl in vitro als auch am Säugetierorganismus von kurzer Dauer, da sie schnell in pharmakologisch unwirksame Metabolite umgewandelt werden. Darüberhinaus ist es nachteilig, daß diese Verbindungen neben der gewünschten physiologischen Wirkung gleichzeitig eine Reihe von unerwünschten physiologischen Nebenwirkungen besitzen, die ihre Verwendung als Arzneimittel stark einschränken.

Es bestand deshalb die Aufgabe, neue Verbindungen mit differenzierten pharmakologischen Wirkungen aufzufinden, die zur Herstellung von Arzneimitteln verwendet werden können. Diese Aufgabe wurde durch die Bereitstellung der Verbindungen der allgemeinen Formel I gelöst.

Es wurde gefunden, daß die Verbindungen der allgemeinen Formel I und ihre physiologisch unbedenklichen Salze bei guter Verträglichkeit wertvolle pharmakologische Eigenschaften besitzen. So zeigen sich beispielsweise Wirkungen auf den Kreislauf, insbesondere blutdrucksenkende Wirkungen sowie Wirkungen auf die Herzkraft (positiv inotrope Wirksamkeit), aber auch thrombozytenaggregationshemmende Wirkungen.

Die Substanzen vermindern z. B. den an der narkotisierten normotonen Ratte gemessenen Blutdruck (Methodik vgl. B.A. Schölkens et al., Prostaglandins and Medicines, Bd. III, 7 - 22, 1979) bei intravenöser Gabe dosisabhängig. Die Abnahme des Blutdruckes (Registrierung über einen in der Arteria femoralis liegenden Katheter, nachgeschaltete SCHRÖDER-Kapsel und ein Quecksilber-U-Rohr auf einem Kymographion) nach Verabreichung der vorliegenden Verbindungen ist auch an der mit Butallylonal narkotisierten Katze bei 10minütiger Dauerinfusion der Substanzen zu beobachten.

Die thrombozytenaggregationshemmende Wirkung läßt sich z. B. in vitro im Aggregationstest nach G.V.R. BORN (Nature 194, 927 - 929, 1962) sowie in vivo durch den Laser-induzierten Thrombosierungstest an Mesenterialgefäßen der Ratte (Methodik in Anlehnung an J. KOVACZ (Microvascular Research 6, 194, 1973) bzw. J. DUHAULT (Arzneimittelforschung/Drug Research 22/10, 1686 - 1690, 1972)) nachweisen.

Die Herzwirkung kann z.B. an narkotisierten oder wachen Hunden, Katzen, Affen oder Minischweinen, aber auch an isolierten Herzpräparationen (z.B. Vorhof, Papillarmuskel oder perfundiertes Ganzherz) von Ratte, Meerschweinchen oder Katze ermittelt werden, z.B. nach Methoden, wie sie in Arzneimittelforschung, Band 31 (I) Nr. 1a (1981), Seiten 141 bis 170, beschrieben sind.

Weiterhin sind die Verbindungen der Formel I zytoprotektiv an verschiedenen Organen des menschlichen oder tierischen Körpers wirksam. So hemmen sie z.B. am Magen die Säuresekretion weit stärker als $PGE_2$ (bei intravenöser Gabe) und schützen die Mucosa gegen den Einfluß von ulcerogen wirkenden Substanzen wie Alkohol, Acetylsalicylsäure oder Taurocholsäure. An der Leber schützen sie gegen toxische Einflüsse, z.B. von $CCl_4$ oder Paracetamol, und erhöhen die Überlebenschancen bei fulminantem Leberversagen. Am Herz schützen sie bei Infarkten vor Nekrosen des Herzgewebes; sie verringern die Infarktgröße und verkürzen die Heilungszeit. Eine Prinzmetal-Angina kann mit den Substanzen der Formel I behandelt werden. Sie zeigen auch am Gehirn günstige Einflüsse, z.B. wenn Ischämien aufgetreten sind.

Außerdem treten bei den 13-Thia-Prostacyclinen der Formel I vasodilatatorische, diuretische, bronchienentkrampfende, die Magensaftsekretion, den Lipidabbau und die Noradrenalin-Freisetzung hemmende, sowie Nasenschleimhaut-abschwellende Eigenschaften auf, die ebenfalls nach hierfür geläufigen Methoden ermittelt werden können. Ferner können die 13-Thia-Prostacycline der allgemeinen Formel I auch die Funktion des corpus luteum, den Eitransport durch den Eileiter, die Nidation und die Fertilität beeinflussen.

Die Verbindungen der allgemeinen Formel I und ihre physiologisch unbedenklichen Salze können daher als Arzneimittelwirkstoffe in der Human- und Veterinärmedizin und auch als Zwischenprodukte zur Herstellung anderer Arzneimittelwirkstoffe verwendet werden.

Gegenstand der Erfindung sind die 13-Thia-Prostacycline der allgemeinen Formel I, in denen A, B, D, m und $R^1$ bis $R^3$ die oben angegebene Bedeutung haben, sowie, falls $R^1$ = H ist, deren physiologisch unbedenkliche Metall- und Ammoniumsalze.

In der allgemeinen Formel I und den anderen Formeln dieser Anmeldung ist eine α-ständige Bindung punktiert und eine ß-ständige Bindung keilförmig ausgezogen eingezeichnet. Bindungen, die α- oder ß-ständig sein können, sind durch eine Wellenlinie gekennzeichnet. Bevorzugt sind Verbindungen, bei denen in der Thioether-Seitenkette die Hydroxygruppe α-ständig und die $C_mH_{2m+1}$-Gruppe ß-ständig ist.
Falls die Bindung ----- für eine Einfachbindung steht, so kann diese α- oder ß-ständig sein. Falls die Bindung ----für eine Doppelbindung steht, so kann diese in der E- oder Z-Konfiguration sein. Bei Verbindungen, in denen A = $-CH_2-$ oder eine Bindung bedeutet, ist die Doppelbindung vorzugsweise in der E-Konfiguration, bei Verbindungen, in denen A = -O- bedeutet, ist die Doppelbindung vorzugsweise in der Z-Konfiguration.

Die Verbindungen der allgemeinen Formel I enthalten 4 asymmetrische C-Atome am carbocyclischen Fünfring. In der Thioether-Seitenkette und auch an der Verknüpfungsstelle der oberen Seitenkette mit dem oberen Ringsystem können weitere Asymmetriezentren auftreten.

Die Verbindungen der allgemeinen Formel I können daher in einer Vielzahl stereoisomerer Formen auftreten.

Gegenstand der Erfindung sind neben den einzelnen Racematen und racemischen Gemischen auch die verschiedenen optisch aktiven Isomeren der allgemeinen Formel I.

Gegenstand der Erfindung ist ferner ein Verfahren zur Herstellung der Verbindungen der allgemeinen Formel I sowie ihrer physiologisch unbedenklichen Metall- und Ammoniumsalze, das dadurch gekennzeichnet ist, daß man

a) eine Verbindung der allgemeinen Formel II

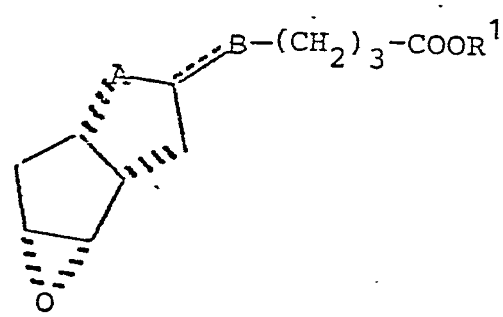

(II)

worin

A, B und $R^1$ die oben angegebenen Bedeutungen haben,

mit einer Verbindung der allgemeinen Formel III

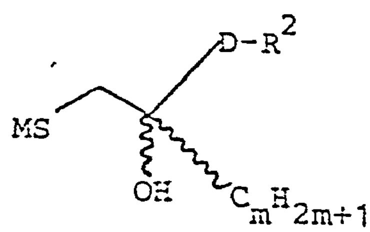

(III)

worin

M H, ein Äquivalent eines Alkali- oder Erdalkalimetallatoms oder ein gegebenenfalls substituiertes Ammoniumion bedeutet,
und

D, m und $R^2$ die oben angegebenen Bedeutungen haben,

umsetzt,

oder daß man

b) in einer Verbindung der allgemeinen Formel IV

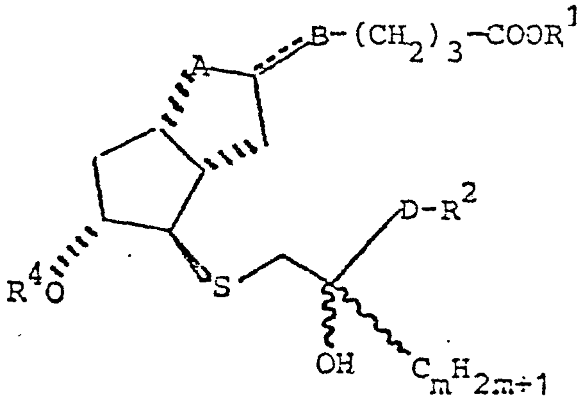

(IV)

worin

$R^4$ eine solvolytisch leicht abspaltbare Schutzgruppe bedeutet, und

A, B, D, m und $R^1$ bis $R^3$ die oben angegebenen Bedeutungen haben,

die Schutzgruppe unter neutralen oder alkalischen Bedingungen abspaltet,

oder daß man

c) aus einer Verbindung der allgemeinen Formel V

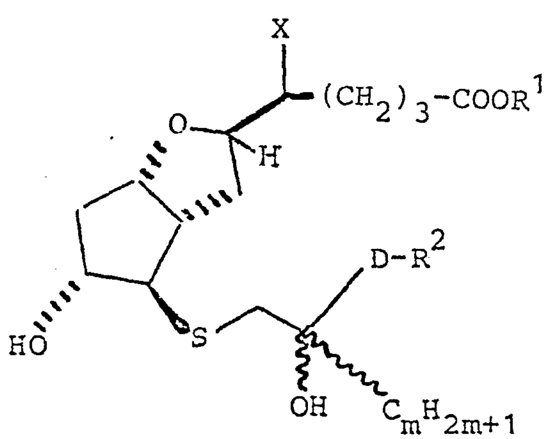

(V)

worin

X Chlor, Brom oder Jod bedeutet, und
D, m und $R^1$ bis $R^3$ die oben angegebenen Bedeutungen haben,

HX abspaltet,

wobei man eine Verbindung der allgemeinen Formel I erhält, in der A -O- und B =CH- bedeutet,

oder daß man

d) eine Verbindung der allgemeinen Formel VI

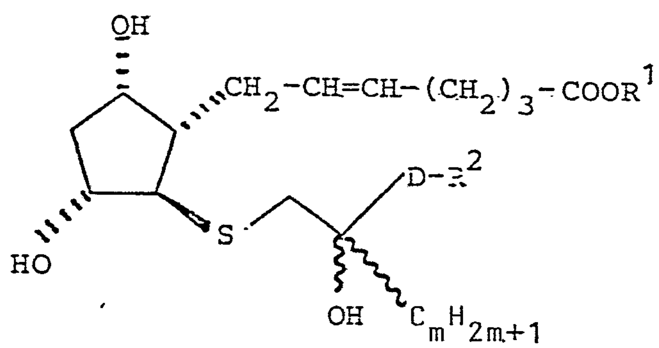

(VI)

worin

D, m und $R^1$ bis $R^3$ die oben angegebenen Bedeutungen haben,

mit einem elektrophilen Reagenz umsetzt und anschließend mit einem Reduktionsmittel behandelt,

wobei man eine Verbindung der allgemeinen Formel I erhält, in der A -O- und B $-CH_2-$ bedeutet,

oder daß man

e) eine Verbindung der allgemeinen Formel VII

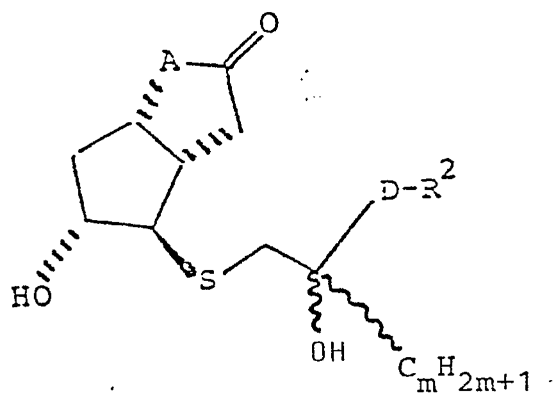

(VII)

worin

A, $-CH_2-$ oder eine Bindung bedeutet und D, m und $R^2$ die oben angegebenen Bedeutungen haben,

mit einem Ylid der allgemeinen Formel VIII

$$(C_6H_5)_3P=CH-(CH_2)_3-COO^{(-)}\ Q^{(+)} \qquad (VIII)$$

worin

$Q^{(+)}$ ein metallisches Kation bedeutet,

umsetzt,

wobei man eine Verbindung der allgemeinen Formel I erhält, in der A $-CH_2-$ oder eine Bindung, B =CH- und $R^1$ H bedeuten,

und/oder daß man

f) eine Verbindung der allgemeinen Formel I mit B = =CH- zu einer Verbindung der allgemeinen Formel I mit B = $-CH_2-$ hydriert

g) und daß man gegebenenfalls eine erhaltene Verbindung der allgemeinen Formel I mit $R^1$ = H verestert zu einer Verbindung der allgemeinen Formel I mit $R^1$ = Alkyl mit 1 - 4 C-Atomen, Aryl mit 6 - 12 C-Atomen oder $-C_6H_4-NH-CO-R^3$ oder eine erhaltene Verbindung der allgemeinen Formel I mit $R^1$ = Alkyl mit 1 - 4 C-Atomen, Aryl mit 6 - 12 C-Atomen oder $-C_6H_4-NH-CO-R^3$ verseift zu einer Verbindung der allgemeinen Formel I mit $R^1$ = H und/oder eine als Racemat erhaltene Verbindung der allgemeinen Formel I in ihre Enantiomeren aufspaltet und/oder eine erhaltene Verbindung der allgemeinen Formel I mit $R^1$ = H durch Umsetzen mit einer Base in eines ihrer physiologisch unbedenklichen Metall- oder Ammoniumsalze überführt.

In der allgemeinen Formel I bedeutet A vorzugsweise -O- oder $-CH_2-$, insbesondere $-CH_2-$, aber auch eine Bindung.

B bedeutet vorzugsweise =CH-, aber auch $-CH_2-$.

Demzufolge sind die Verbindungen ganz besonders bevorzugt, in denen A $-CH_2-$ und B =CH- bedeuten. Auch Verbindungen, in denen A -O- oder eine Bindung und B =CH- bedeuten, sind bevorzugt.

D bedeutet vorzugsweise eine Bindung sowie Alkylen mit 1 - 3 C-Atomen, insbesondere Methylen, aber auch Ethylen, Propylen oder 1-Methyl-ethylen. Wenn D cis-Alkenylen mit 2 - 5 C-Atomen bedeutet, so sind unverzweigte cis-Alkenylen-Gruppen mit 3 - 5 C-Atomen bevorzugt, insbesondere cis-But-2-enylen. D kann aber auch für eine verzweigte cis-Alkenylen-Gruppe stehen, beispielsweise für 1-Methyl-but-2-enylen. Wenn D Alkinylen mit 2 - 5 C-Atomen bedeutet, so sind verzweigte Alkinylen-Gruppen mit 3 - 5 C-Atomen bevorzugt, insbesondere 1-Pentin-1,4-ylen.

m bedeutet vorzugsweise 0 oder 1, ferner aber auch 2 oder 3. Die Gruppe $C_m H_{2m+1}$ steht somit insbesondere für Wasserstoff oder Methyl.

$R^1$ bedeutet insbesondere Wasserstoff, aber auch einen Alkylrest, vorzugsweise einen unverzweigten Alkylrest mit bis zu 4 C-Atomen, wie Methyl, Ethyl, Propyl oder n-Butyl, aber auch einen verzweigten, wie Isopropyl oder tert.-Butyl. Ferner bedeutet $R^1$ einen Arylrest mit 6 - 12 C-Atomen wie Phenyl, Tolyl, Biphenylyl oder Naphthyl. Besonders vorteilhaft bedeutet $R^1$ auch den Rest $-C_6H_4-NH-CO-R^3$, wobei $R^3$ vorzugsweise Phenyl, aber auch $NH_2$ oder $CH_3$ bedeutet.

$R^2$ bedeutet z.B. einen Alkylrest mit 1 - 7 C-Atomen, vorzugsweise einen unverzweigten Alkylrest mit 1 - 7 C-Atomen, wie Methyl, Ethyl, Propyl, Butyl, Pentyl, Hexyl oder Heptyl. $R^2$ kann aber auch ein verzweigter Alkylrest mit 3 - 7 C-Atomen sein, wie beispielsweise 1-Methylpentyl, 1,1-Dimethylpentyl, 2-Methylpentyl, 3-Methylpentyl, 3,3-Dimethylpentyl, 4-Methylpentyl oder 1-Methylhexyl.

Falls $R^2$ einen durch Halogen substituierten Alkylrest mit 1 - 7 C-Atomen bedeutet, so befindet sich der Halogensubstituent, der vorzugsweise Fluor oder Chlor ist, aber auch Brom oder Jod sein kann, insbesondere in der Endstellung. Als Beispiele seien genannt: Chlormethyl, 2-Chlorethyl, 3-Chlorpropyl, 4-Chlorbutyl, 5-Chlorpentyl, 1-Methyl-4-chlorbutyl, 1-Methyl-5-chlorpentyl, 1,1-Dimethyl-4-chlorbutyl; Fluormethyl, 2-Fluorethyl, 3-Fluorpropyl, 4-Fluorbutyl, 5-Fluorpentyl; 4-Brombutyl, 5-Brombutyl.

$R^2$ bedeutet vorzugsweise auch Cycloalkyl mit 5 - 6 C-Atomen, wie Cyclopentyl und insbesondere Cyclohexyl. Ferner kann $R^2$ durch Alkyl mit 1 - 4 C-Atomen, vorzugsweise 1 - 2 C-Atomen substituiertes Cycloalkyl mit 5 - 6 C-Atomen bedeuten, wie beispielsweise 2-Methylcyclohexyl, 3-Methylcyclohexyl, 4-Methylcyclohexyl, 4-Ethylcyclohexyl, 4-Propylcyclohexyl, 4-Isopropylcyclohexyl, 4-Butylcyclohexyl, 4-tert. Butylcyclohexyl; 2-Methylcyclopentyl, 3-Methylcyclopentyl, 3-Ethylcyclopentyl, 3-Propylcyclopentyl, 3-Butylcyclopentyl.

$R^2$ bedeutet auch vorzugsweise Phenyl oder ein-, zwei- oder dreifach durch F, Cl, Br, Alkyl mit 1 - 4 C-Atomen, OH, $OCH_3$, oder $CF_3$ substituiertes Phenyl. Wenn $R^2$ ein substituierter Phenylrest ist, so ist er vorzugsweise einfach substituiert, wobei der Substituent in 2-, 3- oder 4-Stellung zu finden ist.

$R^2$ ist daher vorzugsweise auch Phenyl, 3-Fluorphenyl, 4-Fluorphenyl, 2-Chlorphenyl, 3-Chlorphenyl, 4-Chlorphenyl, 3-Bromphenyl, 4-Bromphenyl, 2-Tolyl, 3-Tolyl, 4-Tolyl, 4-Ethylphenyl, 4-Isopropylphenyl, 4-Butylphenyl, 3-Hydroxyphenyl, 4-Hydroxyphenyl, 3-Methoxyphenyl, 4-Methoxyphenyl, 2-Trifluormethylphenyl, 3-Trifluormethylphenyl, 4-Trifluormethylphenyl, aber auch beispielsweise 2,4-Dichlor-, 3,4-Dichlor-, 2,4-Dimethyl-, 3,4-Dimethyl-, 2,4-Dimethoxy-, 2,3-Dimethoxy-, 2,4,6-Trimethyl- oder 3,4,5-Trimethoxyphenyl.

$R^2$ bedeutet ferner 2-Pyridyl, 3-Pyridyl, 4-Pyridyl, 1-Naphthyl, 2-Naphthyl, 2-Thienyl oder 3-Thienyl.

Falls D Alkylen mit 1 - 3 C-Atomen, insbesondere Methylen bedeutet, steht $R^2$ auch vorzugsweise für Alkoxy mit 1 - 4 C-Atomen, wie Methoxy, Ethoxy, Propyloxy oder Butyloxy; Alkylthio mit 1 - 4 C-Atomen wie Methylthio, Ethylthio, Propylthio oder Butylthio; Phenoxy oder

ein-, zwei- oder dreifach durch F, Cl, Br, Alkyl mit 1 - 4 C-Atomen, OH, $OCH_3$ oder $CF_3$ substituiertes Phenoxy. Wenn $R^2$ ein substituierter Phenoxyrest ist, so ist er vorzugsweise einfach substituiert, wobei der Substituent in 2-Stellung, insbesondere aber in 3- oder 4-Stellung zu finden ist. Beispiele für solche Reste sind Phenoxy, 3-Fluorphenoxy, 4-Fluorphenoxy, 3-Chlorphenoxy, 4-Chlorphenoxy, 3-Bromphenoxy, 4-Bromphenoxy, 2-Methylphenoxy, 3-Methylphenoxy, 4-Methylphenoxy, 4-Ethylphenoxy, 4-Isopropylphenoxy, 4-Butylphenoxy, 3-Hydroxyphenoxy, 4-Hydroxyphenoxy, 3-Methoxyphenoxy, 4-Methoxyphenoxy, 3-Trifluormethylphenoxy, 4-Trifluormethylphenoxy, aber auch beispielsweise 2,4-Dichlor-, 3,4-Dichlor-, 2,4-Dimethyl-, 3,4-Dimethyl-, 2,4-Dimethoxy-, 2,3-Dimethoxy-, 2,4,6-Trimethyl- oder 3,4,5-Trimethoxyphenoxy.

D-$R^2$ bedeutet demnach vorzugsweise Butyl, Pentyl, Hexyl, Heptyl, Octyl, Nonyl, Decyl; 1-Methylpentyl, 1,1-Dimethylpentyl, 2-Methylpentyl, 3-Methylpentyl, 4-Methylpentyl; 4-Chlorbutyl, 4-Chlorpentyl, 5-Chlorpentyl, 1-Methyl-4-chlorbutyl, 1,1-Dimethyl-4-chlorbutyl, 5-Fluorpentyl, 5-Brompentyl; But-2-enyl, Pent-2-enyl, Hex-2-enyl, Pent-3-enyl, Hex-3-enyl, Hex-4-enyl, Hept-4-enyl; Cyclopentyl, Cyclohexyl, 3-Methylcyclohexyl, 4-Methylcyclohexyl, 3-Ethylcyclohexyl, 4-Isopropylcyclohexyl, Cyclohexylmethyl, 2-Cyclohexylethyl, Cyclopentylmethyl; Phenyl, 2-Fluorphenyl, 3-Fluorphenyl, 4-Fluorphenyl, 2-Chlorphenyl, 3-Chlorphenyl, 4-Chlorphenyl, 2-Tolyl, 3-Tolyl, 4-Tolyl, 3-Methoxyphenyl, 4-Methoxyphenyl, 2-Trifluormethylphenyl, 3-Trifluormethylphenyl, 4-Trifluormethylphenyl; Benzyl, 4-Fluorbenzyl, 3-Chlorbenzyl, 4-Chlorbenzyl, 4-Methylbenzyl, 4-Methoxybenzyl, 3-Trifluormethylbenzyl, 4-Trifluormethylbenzyl; 2-Phenylethyl, 2-(3-Chlorphenyl)-ethyl, 2-(3-Trifluormethylphenyl)-ethyl; 4-Pyridyl, 4-Pyridylmethyl, 2-Naphthyl, 2-Naphthylmethyl, 2-Thienyl, 2-Thienylmethyl, 3-Thienyl,

3-Thienylmethyl; Ethoxymethyl, Propyloxymethyl, Butyloxymethyl, 2-Methoxyethyl, 2-Ethoxyethyl, 2-Propyloxyethyl, 3-Methoxypropyl, 3-Ethoxypropyl; Ethylthiomethyl, Propylthiomethyl, Butylthiomethyl, 2-Methylthiomethyl, 2-Ethylthioethyl, 2-Propylthioethyl, 3-Methylthiopropyl, 3-Ethylthiopropyl; Phenoxymethyl, 3-Fluorphenoxymethyl, 4-Fluorphenoxymethyl, 3-Chlorphenoxymethyl, 4-Chlorphenoxymethyl, 4-Methoxyphenoxymethyl, 3-Trifluormethylphenoxymethyl, 4-Trifluormethylphenoxymethyl.

Als physiologisch unbedenkliche Metall- und Ammoniumsalze kommen insbesondere die Natrium-, Kalium-, Magnesium-, Calcium- und Ammoniumsalze in Betracht, ferner substituierte Ammoniumsalze, wie z. B. die Monoethanol- und Triethanolammonium-, Cyclohexylammonium- und Dibenzylethylendiammonium-Salze.

Dementsprechend sind Gegenstand der Erfindung insbesondere diejenigen Verbindungen der allgemeinen Formel I, in denen mindestens einer der genannten Reste eine der vorstehend angegebenen, insbesondere der vorstehend angegebenenen bevorzugten Bedeutungen hat. Einige bevorzugte Gruppen von Verbindungen können durch die folgenden Teilformeln Ia bis Io ausgedrückt werden, die der allgemeinen Formel I entsprechen, worin jedoch

| | | |
|---|---|---|
| in Ia | A | -O- oder $-CH_2-$ und |
| | B | $-CH_2-$ oder =CH- bedeuten; |
| in Ib | m | O oder 1 bedeutet; |
| in Ic | $R^1$ | H, Methyl, Ethyl oder $-C_6H_4-NH-CO-C_6H_5$ bedeutet; |
| in Id | $R^2$ | Alkyl mit 3 - 6 C-Atomen, Cyclohexyl, Phenyl, durch F, Cl, $CH_3$, $OCH_3$ oder $CF_3$ einfach substituiertes Phenyl, oder Naphthyl bedeutet; |

| | | |
|---|---|---|
| in Ie | D | eine Bindung und |
| | $R^2$ | Alkyl mit 3 - 6 C-Atomen, Cyclohexyl, Phenyl, durch F, Cl, $CH_3$, $OCH_3$ oder $CF_3$ einfach substituiertes Phenyl, oder Naphthyl bedeutet; |
| in If | D | Methylen und |
| | $R^2$ | Alkoxy mit 1 - 4 C-Atomen, Alkylthio mit 1 - 4 C-Atomen, Phenoxy oder durch F, Cl oder $CF_3$ einfach substituiertes Phenoxy bedeuten; |
| in Ig | A | -O- oder $-CH_2-$, |
| | B | $-CH_2-$ oder =CH-, |
| | m | O oder 1 und |
| | $R^1$ | H, Methyl, Ethyl oder $-C_6H_4-NH-CO-C_6H_5$ bedeuten; |
| in Ih | A | -O- oder $-CH_2-$, |
| | B | $-CH_2-$ oder =CH-, |
| | m | O oder 1, |
| | $R^1$ | H, Methyl, Ethyl oder $-C_6H_4-NH-CO-C_6H_5$ und |
| | $R^2$ | Alkyl mit 3 - 6 C-Atomen, Cyclohexyl, Phenyl, durch F, Cl, $CH_3$, $OCH_3$ oder $CF_3$ einfach substituiertes Phenyl, oder Naphthyl bedeuten; |
| in Ii | A | -O- oder $-CH_2-$, |
| | B | $-CH_2-$ oder =CH-, |
| | m | O oder 1, |
| | $R^1$ | H, Methyl, Ethyl oder $-C_6H_4-NH-CO-C_6H_5$, |
| | D | eine Bindung und |
| | $R^2$ | Alkyl mit 3 - 6 C-Atomen, Cyclohexyl, Phenyl, durch F, Cl, $CH_3$, $OCH_3$ oder $CF_3$ einfach substituiertes Phenyl, oder Naphthyl bedeutet; |

| | | |
|---|---|---|
| in Ij | A | -O- oder $-CH_2-$, |
| | B | $-CH_2-$ oder =CH-, |
| | m | O oder 1, |
| | $R^1$ | H, Methyl, Ethyl oder $-C_6H_4-NH-CO-C_6H_5$, |
| | D | Methylen und |
| | $R^2$ | Alkoxy mit 1 - 4 C-Atomen, Alkylthio mit 1 - 4 C-Atomen, Phenoxy oder durch F, Cl oder $CF_3$ einfach substituiertes Phenoxy bedeuten; |
| in Ik | A | -O- oder $-CH_2-$ und |
| | B | =CH- bedeuten; |
| in Il | A | -O- oder $-CH_2-$, |
| | B | =CH-, |
| | m | O oder 1, |
| | $R^1$ | H, Methyl, Ethyl oder $-C_6H_4-NH-CO-C_6H_5$ und |
| | $R^2$ | Alkyl mit 3 - 6 C-Atomen, Cyclohexyl, Phenyl, durch F, Cl, $CH_3$, $OCH_3$ oder $CF_3$ einfach substituiertes Phenyl, oder Naphthyl bedeuten; |
| in Im | A | -O- oder $-CH_2-$, |
| | B | =CH-, |
| | m | O oder 1, |
| | $R^1$ | H, Methyl, Ethyl oder $-C_6H_4-NH-CO-C_6H_5$, |
| | D | eine Bindung und |
| | $R^2$ | Alkyl mit 3 - 6 C-Atomen, Cyclohexyl, Phenyl, durch F, Cl, $CH_3$, $OCH_3$ oder $CF_3$ einfach substituiertes Phenyl, oder Naphthyl bedeutet; |
| in In | A | -O- oder $-CH_2-$, |
| | B | =CH-, |
| | m | O oder 1, |
| | $R^1$ | H, Methyl, Ethyl oder $-C_6H_4-NH-CO-C_6H_5$, |
| | D | Methylen und |
| | $R^2$ | Alkoxy mit 1 - 4 C-Atomen, Alkylthio mit 1 - 4 C-Atomen, Phenoxy oder durch F, Cl oder $CF_3$ einfach substituiertes Phenoxy bedeuten; |

in Io A -O- oder $-CH_2-$,
B =CH-,
m 0 oder 1,
$R^1$ H, Methyl, Ethyl oder $-C_6H_4-NH-CO-C_6H_5$ und
$D-R^2$ Pentyl, Hexyl, Cyclohexyl, Phenyl, Phenylmethyl, 4-Fluorphenyl, 2-Chlorphenyl, 3-Chlorphenyl, 4-Chlorphenyl, Ethoxymethyl, Ethylthiomethyl, Butyloxymethyl, Butylthiomethyl, Phenoxymethyl, 3-Chlorphenoxymethyl, 4-Chlorphenoxymethyl, 3-Trifluormethylphenoxymethyl, Pent-2-enyl oder Hex-2-enyl bedeuten.

Die Herstellung der Verbindungen der allgemeinen Formel I erfolgt im übrigen nach an sich bekannten Methoden, wie sie in der Literatur (z. B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der allgemeinen Formeln II bis VIII können gewünschtenfalls auch in situ gebildet werden, derart, daß man sie aus dem Reaktionsgemisch nicht isoliert, sondern sofort weiter zu den Verbindungen der allgemeinen Formel I umsetzt.

Die einzelnen Verfahrensweisen werden im folgenden näher erläutert:

a) Die Umsetzung einer Verbindung der Formel II mit einem Thiol oder Thiolat der Formel III erfolgt in der Regel in Gegenwart eines basischen Kataly-

0084775

sators und in Anwesenheit oder Abwesenheit eines inerten Lösungsmittels bei Temperaturen zwischen etwa -20 und +100°, vorzugsweise zwischen 20 und 70°.

Als Lösungsmittel eignen sich vorzugsweise Alkohole wie Methanol, Ethanol, Propanol, Isopropanol, Butanol, tert.-Butylalkohol, Amylalkohol, 2-Methoxyethanol oder 2-Ethoxyethanol; Ether wie Diethylether, Tetrahydrofuran (THF), Dioxan oder Ethylenglykoldimethylether; chlorierte Kohlenwasserstoffe wie Methylenchlorid oder Chloroform oder auch Wasser.

Geeignete basische Katalysatoren sind z. B. Alkalimetall- oder Erdalkalimetallhydroxide wie NaOH, KOH oder $Ca(OH)_2$; Alkalimetallalkoholate, wie $NaOCH_3$, $NaOC_2H_5$ oder KO-tert.-$C_4H_9$; basische Salze, vorzugsweise Carbonate oder Acetate wie $K_2CO_3$ oder $NaOCOCH_3$; Ammoniak; Amine, vorzugsweise sekundäre oder tertiäre Amine wie Triethylamin, Diisopropylamin, Dicyclohexylamin, Dimethylanilin, Piperidin, 2,6-Dimethylpiperidin, 2,2,6,6-Tetramethylpiperidin, Pyrrolidin, Pyridin, Chinolin, Diazabicyclo-[2.2.2]-octan oder Diazabicyclo[3.4.0]-nonen; aber auch primäre Amine wie tert.-Butylamin oder Cyclohexylamin; oder quartäre Ammoniumhydroxide wie Tetramethylammoniumhydroxid oder insbesondere Benzyltrimethylammoniumhydroxid. Man kann auch eines der genannten Amine, insbesondere ein sekundäres oder tertiäres Amin, gleichzeitig als Lösungsmittel verwenden und so in Abwesenheit eines der genannten inerten Lösungsmittel arbeiten.

Besonders zweckmäßig arbeitet man unter einer Inertgasatmosphäre, beispielsweise unter Stickstoff.

b) Verbindungen der Formel IV, bei denen die Hydroxygruppe in 3-Stellung am Ringsystem durch eine solvolytisch leicht abspaltbare Gruppe geschützt ist und die sonst der Formel I entsprechen, können unter neutralen oder alkalischen Bedingungen in Verbindungen der Formel I überführt werden. Bei der Gruppe $-OR^4$ handelt es sich um eine beliebige Ester- oder Ethergruppe, die durch Solvolyse in die Hydroxygruppe überführt werden kann. Beispiele für solche Estergruppen sind die, in denen der Acylrest sich von einer aliphatischen Monocarbonsäure mit 1 -6 C-Atomen, einer aromatischen Monocarbonsäure mit 7 - 13 C-Atomen, Phosphorsäure, Kohlensäure oder einer aliphatischen Dicarbonsäure mit bis zu 6 C-Atomen ableitet. Solche Estergruppen können aus Säureanhydriden und Säurehalogeniden, insbesondere Chloriden oder Bromiden, gebildet werden, wobei die aliphatischen Carbonsäureanhydride, zum Beispiel Acetanhydrid und Capronsäureanhydrid; aromatischen Carbonsäureanhydride, zum Beispiel Benzoesäureanhydrid; aliphatischen Dicarbonsäureanhydride, zum Beispiel Bernsteinsäureanhydrid, und Chlorameisensäureester, zum Beispiel Chlorameisensäuretrichlorethylester bevorzugt sind. Eine besonders geeignete Etherschutzgruppe ist beispielsweise der Tetrahydropyranylether oder der 4-Methoxy-5,6-dihydro-2H-pyranylether. Andere solche Ether sind Arylmethylether, wie Benzyl-, Benzhydryl- oder Tritylether, oder aliphatische (α-Alkoxy)alkylether mit bis zu 6 C-Atomen, zum Beispiel Methoxymethyl- oder Allylether oder Trialkylsilylether wie Trimethylsilylether oder Dimethyl-tert.butyl-silylether.

Als solvolysierende Mittel verwendet man vorzugsweise hydrolysierende Mittel wie Wasser oder Wasser im Gemisch mit organischen Lösungsmitteln, in Abwesenheit oder vorzugsweise in Anwesenheit eines basischen Katalysators. Als organische Lösungsmittel kommen z. B. in Frage Alkohole wie Methanol, Ethanol, Propanol, Isopropanol, Butanol, tert.-Butylalkohol, Amylalkohol, 2-Methoxyethanol oder 2-Ethoxyethanol; Ether wie Diethylether, THF, Dioxan oder Ethylenglykoldimethylether, Säuren wie Ameisensäure, Essigsäure, Propionsäure oder Buttersäure; Ester wie Ethylacetat oder Butylacetat; Ketone wie Aceton; Amide wie Dimethylformamid (DMF) oder Phosphorsäurehexamethyltriamid; Nitrile wie Acetonitril; Sulfoxide wie Dimethylsulfoxid (DMSO); Sulfone wie Tetrahydrothiophen-S,S-dioxid; sowie Gemische dieser Lösungsmittel.

Als basische Katalysatoren verwendet man zweckmäßig Alkalimetall- oder Erdalkalimetallhydroxide wie Natrium-, Kalium- oder Calciumhydroxid, oder basische Salze, wie Natrium- oder Kaliumcarbonat. Auch organische Basen, beispielsweise Ethyl-, Diethyl-, Triethyl-, Isopropyl-, n-Butyl- oder Tri-n-butylamin, Ethanolamin, Triethanolamin, Cyclohexylamin, Dimethylanilin, Pyrrolidin, Piperidin, Morpholin, Pyridin, α-Picolin oder Chinolin; oder quartäre Ammoniumhydroxide, wie z. B. Tetramethylammoniumhydroxid oder Benzyltrimethylammoniumhydroxid können als basische Katalysatoren verwendet werden. Ein Überschuß des Katalysators kann auch an Stelle eines Lösungsmittels verwendet werden.

Die Abspaltung der Schutzgruppe wird bei Temperaturen zwischen -5 und 80°, vorzugsweise bei Raumtemperatur ausgeführt. Die Solvolysezeiten liegen zwischen 1 und 48 Stunden.

c) Die Abspaltung von HX aus Verbindungen der Formel V unter Bildung von Verbindungen der Formel I, in denen A -O- und B =CH- bedeutet, verläuft unter der Einwirkung von Basen in Gegenwart oder Abwesenheit eines Lösungsmittels.

Als Basen kommen sowohl anorganische wie organische in Betracht, wie z. B. Alkalimetallhydroxide oder -carbonate, Alkoholate wie z. B. Natriummethylat oder Kalium tert.butylat, Amine wie z. B. Triethylamin, 4-Dimethylaminopyridin, Dicyclohexylethylamin oder 1,4-Diazabicyclo[2,2,2]octan, Amidine wie z. B. 1,5-Diazabicyclo[3,4,0]nonen-5 (DBN) oder 1,5-Diazabicyclo[5,4,0]undecen-5 (DBU).

Als Lösungsmittel eignen sich beispielsweise Alkohole, wie Methanol, Ethanol, Propanol, Butanol; Kohlenwasserstoffe wie Benzol oder Toluol. Man arbeitet bei Temperaturen zwischen 20° und 120°; die Reaktionszeiten liegen in der Regel zwischen 0,5 und 10 Stunden. Zweckmäßig arbeitet man unter einer Schutzgasatmosphäre.

d) Verbindungen der Formel I, in denen A -O- und B $-CH_2-$ bedeutet, erhält man vorzugsweise durch Cyclisierung von Verbindungen der Formel VI und anschließende Reduktion, wie es beispielsweise in J.Chem. Res. 2444 (1979) beschrieben wird. Für die Cyclisierung von Verbindungen der Formel VI eignen sich elektrophile Reagenzien, die mit γ-Hydroxyolefinen unter Ringschluß zu Tetrahydrofuran-Derivaten reagieren, wie z. B. Brom, Jod; Jodchlorid;

Quecksilbersalze wie Quecksilber(II)acetat, Quecksilbersulfat oder Quecksilbernitrat; Thalliumsalze wie Thalliumnitrat. Die Reaktion wird zweckmäßig in einem inerten Lösungsmittel, wie z. B. Wasser, Methanol, Ethanol, Propanol, Diethylether, THF oder 1,2-Dimethoxyethan durchgeführt. Es können auch heterogene oder homogene Lösungsmittelgemische verwendet werden. Die Reaktion kann bei Temperaturen zwischen 0 und 70° durchgeführt werden, vorzugsweise bei 10 bis 40°. Die Reaktionszeit beträgt in der Regel 1 bis 12 Stunden.

Das erhaltene cyclisierte Produkt wird anschließend, vorzugsweise ohne Isolierung aus dem Reaktionsgemisch, mit einem Reduktionsmittel behandelt. Als Reduktionsmittel eignen sich z. B. Borhydrid-Reduktionsmittel wie Natriumborhydrid, Kaliumborhydrid, Lithiumborhydrid, Calciumborhydrid, Magnesiumborhydrid; komplexe Trialkylborhydride wie Lithiumthexyllimonylborhydrid; Natriumtrialkoxyborhydride, wie Natriumtrimethoxyborhydrid; ferner beispielsweise Tributylzinnhydrid. Man arbeitet zweckmäßig in einem inerten Lösungsmittel, beispielsweise einem der oben genannten Lösungsmitel, gegebenenfalls unter Zusatz einer Base, wie z. B. einem Alkalimetallhydroxid. Die Reaktionstemperatur liegt zwischen -20 und 40°, vorzugsweise bei Raumtemperatur. Die Reaktionszeiten liegen meist zwischen 15 Minuten und 6 Stunden.

e) Die Umsetzung eines Bicycloketons der Formel VII, worin A $-CH_2-$ oder eine Bindung bedeutet, mit einem (4-Carboxybutyliden)-triphenylphosphoran der Formel VIII zu einer Verbindung der Formel I, worin A $-CH_2-$ oder eine Bindung, B =CH- und $R^1$ H bedeuten, erfolgt unter den für eine Wittig-Reaktion üblichen Reaktionsbedingungen (vgl. beispielsweise Organic Reactions, 14, 3. Kapitel (1965), John Wiley & Sons, Inc., oder Tetrahedron Letters 3743 (1978)).

Die Reaktion wird zweckmäßigerweise in einem inerten organischen Lösungsmittel durchgeführt; geeignet sind Kohlenwasserstoffe wie Cyclohexan, Toluol, Xylol und insbesondere Benzol; ferner Acetonitril; Ether wie Diethylether, Diisopropylether, 1,2-Dimethoxyethan und insbesondere THF; oder auch DMSO. Man arbeitet in Gegenwart einer starken Base wie beispielsweise eines Alkalimethallhydrids wie NaH, einer Lithiumalkylverbindung wie Butyllithium oder eines Alkalimetallalkoholats wie Kaliumtert.butylat. Die Reaktionstemperaturen liegen üblicherweise zwischen 10 und 80°, vorzugsweise bei Raumtemperatur. Besonders zweckmäßig ist es, unter einer Inertgasatmosphäre, beispielsweise unter Stickstoff, zu arbeiten.

f) Eine Verbindung der Formel I mit B $-CH_2-$ kann ferner aus einer Verbindung der Formel I mit B =CH- durch Hydrierung, insbesondere durch katalytische Hydrierung erhalten werden.

Für katalytische Hydrierungen eignen sich beispielsweise Edelmetall-, Nickel- oder Kobaltkatalysatoren, ferner auch Mischkatalysatoren wie Kupferchromoxid. Als Edelmetalle kommen in erster Linie Platin und Palladium in Betracht, die auf Trägern (z. B. auf Kohle, Calciumcarbonat oder Strontiumcarbonat),

als Oxide (z. B. Platinoxid) oder in feinteiliger Form vorliegen können. Nickel- und Kobaltkatalysatoren werden zweckmäßig als Raney-Metalle eingesetzt. Man kann zweckmäßig bei Drucken zwischen etwa 1 und 200 bar und bei Temperaturen zwischen etwa -80 und +150°, vorzugsweise bei Raumtemperatur hydrieren. Die Hydrierung erfolgt in Gegenwart eines inerten Lösungsmittels, z. B. eine Alkohols wie Methanol, Ethanol oder Isopropanol, einer Carbonsäure wie Essigsäure, eines Esters wie Ethylacetat, eines Ethers wie THF oder Dioxan, eines Ketons wie Aceton. Man kann auch Lösungsmittelgemische verwenden, z. B. auch Wasser enthaltende Gemische.

g) Eine Säure der Formel I ($R^1$ = H) kann durch Umsetzen mit einem entsprechenden veresternden Mittel in einen Ester der Formel I ($R^1$ = Alkyl mit 1 - 4 C-Atomen, Aryl mit 6 - 12 C-Atomen oder $-C_6H_4-NH-CO-R^3$) überführt werden. Veresternde Mittel sind beispielsweise Alkohole mit bis zu 4 C-Atomen, vorzugsweise in Gegenwart einer anorganischen oder organischen Säure, wie HCl, HBr, HJ, $H_2SO_4$, $H_3PO_4$, Trifluoressigsäure, einer Sulfonsäure wie Benzolsulfonsäure oder p-Toluolsulfonsäure, oder eines sauren Ionenaustauschers; Diazoalkane mit bis zu 4 C-Atomen, vorzugsweise Diazomethan; Olefine (z. B. Isobutylen), vorzugsweise in Gegenwart von sauren Katalysatoren (z. B. $ZnCl_2$, $BF_3$, $H_2SO_4$, Arylsulfonsäure, Pyrophosphorsäure, Borsäure, Oxalsäure); Alkylhalogenide mit bis zu 4 C-Atomen, vorzugsweise Bromide, wie Ethyl-, Propyl-, Isopropyl- oder Butylbromid, aber auch die entsprechenden -chloride oder -jodide; Carbonsäure- oder Sulfonsäurealkylester, wobei der Säurerest beliebig sein kann und der Alkylrest bis zu 4 C-Atome enthält, vorzugsweise Methyl-, Ethyl-, Propyl-, Isopropyl- oder Butylacetat, -formiat, -methylsulfonat, -ethylsulfonat oder -p-toluolsulfonat; und insbesondere auch Dialkylschwefelsäureester mit bis zu 4 C-Atomen, wie Dimethylsulfat oder Diethylsulfat.

Die Veresterung erfolgt zweckmäßig in einem inerten, vorzugsweise wasserfreien Lösungsmittel, beispielsweise einem Ether wie Diethylether oder THF, einem Alkohol, vorzugsweise einem der genannten Alkohole mit bis zu 4 C-Atomen oder auch in einem Kohlenwasserstoff, wie Petrolether, Hexan, Benzol oder Toluol, oder in Gemischen dieser Lösungsmittel bei Temperaturen zwischen -10° und 40°, vorzugsweise bei Raumtemperatur. Die Reaktionszeiten liegen in der Regel zwischen 30 Minuten und 20 Stunden.

Aromatische Ester der Formel I ($R^1$ = Aryl mit 6 - 12 C-Atomen oder $-C_6H_4-NH-CO-R^3$) können aus Säuren der Formel I ($R^1 = H$) durch Umsetzen mit einem Phenol (wie beispielsweise Phenol, m-Kresol, p-Kresol, p-Ethylphenol, α-Naphthol, ß-Naphthol, p-Phenylphenol) oder einer Verbindung der Formel $HO-C_6H_4-NH-CO-R^3$ (worin $R^3$ die oben angegebene Bedeutung hat) hergestellt werden. Die letztgenannten Verbindungen sind bekannt, beispielsweise aus der DE-OS 24 53 271 oder der DE-OS 26 44 972.

Die Veresterung einer Säure der Formel I ($R^1 = H$) mit einem Phenol kann nach an sich bekannten Methoden durchgeführt werden; vorzugsweise arbeitet man in Gegenwart eines wasserbindenden Mittels, z. B. eines Carbodiimids, wie Dicyclohexylcarbodiimid, und in einem inerten organischen Lösungsmittel, vorzugsweise einem Ether wie Diethylether, 1,2-Dimethoxyethan, THF oder Dioxan; oder einem Halogenkohlenwasserstoff wie Methylenchlorid oder 1,2-Dichlorethan; oder in Gemischen dieser Lösungsmittel mit DMF. Die Reaktionstemperaturen liegen beispielsweise etwa zwischen -20° und 100°.

Ester der Formel I können durch Hydrolyse in Säuren der Formel I umgewandelt werden. Bevorzugt ist die basische Hydrolyse. Man arbeitet vorzugsweise in wässerigen Medien, beispielsweise in Gemischen von Wasser mit Alkoholen, vorzugsweise niederen Alkanolen, wie Methanol oder Ethanol, oder mit Ethern, wie Ethylenglykolmonomethylether, Ethylenglykoldimethylether, THF oder Dioxan bei Temperaturen zwischen 0° und 40°, vorzugsweise bei Raumtemperatur. Die Reaktionszeiten betragen ungefähr 1 Stunde bis 12 Stunden.

Die Verbindungen der Formel I werden meist als Gemische verschiedener stereoisomerer Formen erhalten, d. h. in der Regel als Gemische von Racematen. Racemate können aus den Racematgemischen isoliert und rein erhalten werden, beispielsweise durch Umkristallisieren der Verbindungen selbst oder von gut kristallisierenden Derivaten, insbesondere aber mit Hilfe chromatographischer Methoden, wobei sowohl adsorptionschromatographische oder verteilungschromatographische Methoden als auch Mischformen in Frage kommen.

Die Racemate können nach bekannten Methoden, wie sie in der Literatur angegeben sind, in ihre optischen Antipoden getrennt werden. Die Methode der chemischen Trennung wird bevorzugt.

So kann man eine optisch aktive Base mit der Carboxylgruppe einer Verbindung der Formel I umsetzen. Zum Beispiel kann man die diastereomeren Salze mit optisch aktiven Aminen, wie Chinin, Cinchonidin, Brucin, Cinchonin, Hydroxyhydrindamin, Morphin, 1-Phenylethylamin, 1-Naphthylethylamin, Phenyloxynaphthylmethylamin, Chinidin, Strychnin, basischen Aminosäuren, wie Lysin, Arginin; Aminosäureestern bilden. In ähnlicher Weise lassen sich Ester-Diastereomere durch Veresterung von

Carbonsäuren der Formel ($R^1$ = H) mit optisch aktiven Alkoholen, wie Borneol, Menthol, Octanol-2, herstellen. Die anfallenden diastereomeren Salze bzw. Ester werden durch Kristallisation getrennt und die optisch aktiven Verbindungen aus dem Gemisch in Freiheit gesetzt.

Aber auch die anderen in den Verbindungen der Formel I vorhandenen funktionellen Gruppe können zur Bildung von Diastereomeren herangezogen werden. So kann man z. B. OH-Gruppen mit optisch aktiven Säuren wie (+)- und (-)-Weinsäure oder Camphersäure verestern und aus diesen Derivaten die reinen Enantiomeren gewinnen.

Weiterhin ist es natürlich möglich, optisch aktive Verbindungen nach den beschriebenen Methoden zu erhalten, indem man Ausgangsstoffe verwendet, die bereits optisch aktiv sind.

Ferner kann man die freien Säuren der Formel I ($R^1$ = H) durch Umsetzung mit einer Base in eines ihrer physiologisch unbedenklicher Metall- oder Ammoniumsalze überführen.

Die Ausgangsverbindungen zur Herstellung der erfindungsgemäßen Verbindungen der Formel I nach den vorstehend beschriebenen Methoden sind teils bekannt, zum größten Teil jedoch neu. Neue Ausgangsverbindungen können aus bekannten Verbindungen in Analogie zu bekannten Verfahren hergestellt werden, beispielsweise wie folgt:

Die Verbindungen der Formel II, in denen A, B und $R^1$ die vorstehend angegebenen, insbesondere die als bevorzugt genannten Bedeutungen haben, können analog

den vorstehend unter c) bis f) beschriebenen Verfahren hergestellt werden, wobei man jedoch von Verbindungen ausgeht, die am carbocyclischen Fünfring eine Epoxygruppe tragen, beispielsweise:

1α-Hydroxy-2α-(6-carboxy-hex-2-enyl)-3α,4α-epoxy-cyclopentan, das aus Synth. Comm. 4 (6), 317 (1974) bekannt ist, oder dessen Derivate werden mit Hilfe eines geeigneten elektrophilen Reagenzes, wie beispielsweise $KJ_3$, cyclisiert, und anschließend wird analog der vorstehend beschriebenen Verfahrensvariante c) unter Einwirkung von Basen Halogenwasserstoff, beispielsweise HJ, abgespalten. Man erhält so Verbindungen der Formel II mit A = -O- und B = =CH-.

Verbindungen der Formel II mit A = -O- und B = $-CH_2-$ erhält man z. B. aus 1α-Hydroxy-2α-(6-carboxy-hex-2-enyl)-3α,4α-epoxy-cyclopentan durch Umsetzen mit einem elektrophilen Reagenz und anschließende Reduktion analog der oben unter d) beschriebenen Verfahrensvariante.

Ferner erhält man Verbindungen der Formel II, in denen A = $-CH_2-$ oder eine Bindung und B = =CH- bedeuten, beispielsweise durch Umsetzen eines 2α,3α-Epoxy-bicyclo((3.2.0)heptan-6-ons (bekannt aus DE-OS 28 00 929) bzw. eines 2α,3α-Epoxy-bicyclo-(3.3.0)octan-7-ons erhältlic durch Epoxidierung eines Bicyclo(3.3.0)oct-2-en-7-ons, siehe JACS 74, 2278 (1952)) mit einem (4-Carboxybutyliden)-triphenyl-phosphoran unter den für eine Wittig-Reaktion üblichen Reaktionsbedingungen.

Verbindungen der Formel II mit B= $-CH_2-$ sind beispielsweise auch erhältlich durch Hydrierung von Verbindungen der Formel II mit B= =CH-.

In den Verbindungen der Formel III haben die Reste D, m und $R^2$ die vorstehend angegebenen, insbesondere die als bevorzugt genannten Bedeutungen. Es handelt sich bei den Verbindungen der Formel III um 2-Hydroxythiole oder deren Alkalimetall-, Erdalkalimetall- oder gegebenenfalls substituierte Ammoniumsalze. Die meisten Thiole der Formel III sind bekannt, beispielsweise aus der DE-OS 22 56 537 der DE-OS 24 22 924 und der DE-OS 26 44 972. Neue Verbindungen der Formel III können aus bekannten Verbindungen in Analogie zu bekannten Verfahren hergestellt werden, beispielsweise aus den entsprechenden Oxiranen durch Umsetzen mit $H_2S$ und gegebenenfalls anschließende Überführung in ihre Alkalimetall-, Erdalkalimetall- oder gegebenenfalls substituierte Ammoniumsalze. Ebenso können die Oxirane direkt mit Alkalimetall-, Erdalkalimetall- oder Ammoniumhydrogensulfiden umgesetzt werden, wobei dann direkt die Verbindungen der Formel III mit M ungleich H erhalten werden.

Die Verbindungen der Formel IV können nach den Verfahren hergestellt werden, nach denen auch die Verbindungen der Formel I erhältlich sind, wobei man allerdings von Vorprodukten ausgeht, in denen die Hydroxygruppe in 3-Stellung am Ringsystem in geschützter Form vorliegt bzw. in einer Vorstufe die Schutzgruppe eingeführt wurde.

Verbindungen der Formel V erhält man z. B. durch Cyclisierung von Verbindungen der Formel VI (worin die Substituenten die oben angegebenen Bedeutungen haben) mit einem elektrophilen Reagenz. Verbindungen der Formel VI sind aus den deutschen Offenlegungsschriften 24 22 924 und 25 50 004 bekannt und können nach den dort angegebenen Verfahren erhalten werden. Als elektrophile Reagenzien sind z. B. Jod, Jod-

chlorid, $KJ_3$ oder N-Bromimide wie N-Bromsuccinimid, N-Bromcampherimid oder 1,3-Dibrom-5,5-dimethylhydantoin geeignet. Die Reaktion wird vorzugsweise in einem inerten Lösungsmittel, wie z. B. Wasser, Methylenchlorid, Chloroform, Diethylether, THF oder 1,2-Dimethoxyethan durchgeführt. Es können auch heterogene oder homogene Lösungsmittelgemische verwendet werden. Die Reaktion kann bei Temperaturen zwischen -70 und +30° durchgeführt werden, gegebenenfalls in Gegenwart eines säurebindenden Mittels wie z. B. Calciumcarbonat, Natriumcarbonat oder Natriumhydrogencarbonat.

Die Verbindungen der Formel VI sind zum größten Teil aus den deutschen Offenlegungsschriften 24 22 924 und 25 50 004 bekannt. Neue Verbindungen der Formel VI sind analog den dort beschriebenen Verfahren erhältlich.

Die Verbindungen der Formel VII sind z. B. erhältlich durch Umsetzen von 2-Brom-3-hydroxy-bicyclo-(3.2.0)heptan-6-on (beschrieben in J.C.S.Chem.Comm.1974, 948) mit einem Thiol der Formel III unter basischen Reaktionsbedingungen wie bereits unter Verfahrensvariante a) beschrieben, wobei man eine Verbindung der Formel VII erhält, in der A eine Bindung bedeutet. Verbindungen der Formel VII mit A= $-CH_2-$ erhält man aus Verbindungen der Formel VII, in denen A eine Bindung bedeutet, durch Umsetzen mit $CH_2N_2$ unter den für eine Arndt-Eistert-Reaktion üblichen Reaktionsbedingungen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger- oder

Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z. B. orale) oder parenterale Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Gegenstand der Erfindung ist ferner die Verwendung der Verbindungen der Formel I bei der Bekämpfung von

Krankheiten, insbesondere von allen Formen der Hypertonie sowie von Herzinsuffizienz, ferner zur Beeinflussung der Thrombozytenfunktion im Sinne einer Aggregations-(Adhäsions-)Hemmung.

Gegenstand der Erfindung ist auch die Verwendung der Verbindungen der Formel I bei der therapeutischen Behandlung des menschlichen oder tierischen Körpers. Dabei werden die erfindungsgemäßen Substanzen in der Regel in Analogie zu bekannten, im Handel befindlichen Präparaten ähnlicher Indikation verabreicht, vorzugsweise in Dosierungen zwischen etwa 0,1 und 100 mg, insbesondere zwischen 0,5 und 50 mg pro Dosierungseinheit. Die tägliche Dosierung liegt vorzugsweise zwischen etwa 0,01 und 1 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt. Im Vergleich zu den bisher zur Therapie der Herzinsuffizienz verwendeten Digitalis-Glykosiden zeichnen sich die Verbindungen der Formel I durch verbesserte therapeutische Breite und periphere Entlastung aus.

Vor- und nachstehend sind alle Temperaturen in $^{o}C$ angegeben.

IR-Spektren (IR) wurden durch Angabe der Hauptbanden charakterisiert (als Film).

Die NMR-Spektren (NMR) wurden in $CDCl_3$ gegen Tetramethylsilan gemessen und durch δ-Angabe der Signale in ppm charakterisiert; dabei bedeuten m = Multiplett, q = Quartett, t = Triplett, d = Duplett und s = Singulett.

Alle nachstehend genannten Bicycloverbindungen besitzen die 1ßH, 5ßH-Konfiguration.

"Übliche Aufarbeitung" bedeutet: man dampft ein, gibt Wasser bzw. wässrige Säure und ein organisches Lösungsmittel zu, trennt ab, trocknet die organische Phase mit $MgSO_4$, dampft ein und reinigt durch Chromatographie an Kieselgel.

Beispiel 1

Man löst 22,2 g 5E-(2α,3α-Epoxy-bicyclo(3.3.0)octyliden-7)-pentansäure und 44 g 2α-Hydroxy-3-m-chlorphenoxy-propanthiol unter Rühren und Stickstoff-Atmosphäre in 150 ml THF, versetzt mit 84 g einer 40 %igen methanolischen Benzyltrimethylammoniumhydroxid-Lösung und kocht 5 Stunden. Nach üblicher Aufarbeitung (Ethylacetat/1 n Salzsäure) erhält man 5E-(2ß-(2α-Hydroxy-3-m-chlorphenoxy-propylthio)-3α-hydroxy-bicyclo(3.3.0)octyliden-7)-pentansäure. NMR: 7,15 - 7,25 (m); 5,28 (t); 4,15 (q); 3,15 - 3,25; 2,66 - 2,78 (m).

Beispiele 2 bis 40

Analog Beispiel 1 sind aus den entsprechenden 2α,3α-Epoxiden der Formel II mit den entsprechenden Thiolen der Formel III erhältlich:

2. 5E-(2ß-(2α-Hydroxy-heptylthio)-3α-hydroxy-bicyclo(3.3.0)octyliden-7)-pentansäure. NMR: 5,26 (t); 3,9 (q); 2,85 ; 3,05 (2d); 0,85 (t).

3. 5E-(2ß-(2α-Hydroxy-2ß-methyl-heptylthio)-3α-hydroxy-bicyclo(3.3.0)octyliden-7)-pentansäure. NMR: 5,3 (t); 3,85 (q); 2,8 (q); 1,25 (s); 0,9 (t).

4. 5E-(2ß-(2α-Hydroxy-octylthio)-3α-hydroxy-bicyclo-(3.3.0)octyliden-7)-pentansäure. NMR: 5,28 (t); 3,6 - 4,0 (m); 2,85; 3,0 (2 α); 0,85 (t).

5. 5E-(2ß-(2α-Hydroxy-2ß-methyl-octylthio)-3α-hydroxy-bicyclo(3.3.0)octyliden-7)-pentansäure. NMR:5,3 (t); 3,7 - 4,2 (m); 2,8 -2,9 (m); 1,23 (s); 0,9 (t).

6. 5E-(2ß-(2α-Hydroxy-2-cyclohexyl-ethylthio)-3α-hydroxy-bicyclo(3.3.0)octyliden-7)-pentansäure. NMR: 5,3 (t); 3,85 (q); 3,5 (q); 2,8 (d).

7. 5E-(2ß-(2α-Hydroxy-2-phenyl-ethylthio)-3α-hydroxy-bicyclo(3.3.0)octyliden-7)-pentansäure. NMR: 7,35 (s); 5,35 (t); 4,8 (2d); 3,7 - 4,1 (m); 2,7 - 3,2 (m); 1,2 - 2,5 (m).

8. 5E-(2ß-(2-Hydroxy-2-methyl-2-phenyl-ethylthio)-3α-hydroxy-bicyclo(3.3.0)octyliden-7)-pentansäure. NMR: 7,3 (s); 5,22 (t); 3,8 (t); 2,8 (s); 1,22 (s).

9. 5E-(2ß-(2α-Hydroxy-3-phenyl-propylthio)-3α-hydroxy-bicyclo(3.3.0)octyliden-7)-pentansäure. NMR: 7,3 (s); 5,2 (t); 3,8 - 4,2 (m).

10. 5E-(2ß-(2α-Hydroxy-2-o-chlorphenyl-ethylthio)-3α-hydroxy-bicyclo(3.3.0)octyliden-7)-pentansäure. NMR: 7,45 - 7,6 (m); 7,1 - 7,3 (m); 5,20 (t); 5,10 (2 d); 3,88 (q).

11. 5E-(2ß-(2α-Hydroxy-2-m-chlorphenyl-ethylthio)-3α-hydroxy-bicyclo(3.3.0)octyliden-7)-pentansäure. NMR: 7,35 (s), 7,22 (m); 5,28 (t); 4,7 - 4,8 (m); 3,9 (q).

12. 5E-(2ß-(2α-Hydroxy-2-p-chlorphenyl-ethylthio)-3α-hydroxy-bicyclo(3.3.0)octyliden-7)-pentansäure. NMR: 7,3 (d); 4,25 (t); 2,6 - 3,0 (m).

13. 5E-(2ß-(2α-Hydroxy-2-m-trifluormethylphenyl-ethylthio)-3α-hydroxy-bicyclo(3.3.0)octyliden-7)-pentansäure. NMR: 7,3 - 7,6 (m); 5,3 (t); 4,9 (t); 3,9 (q); 2,9 (2d).

14. 5E-(2ß-(2α-Hydroxy-2-(2-naphthyl)-ethylthio)-3α-hydroxy-bicyclo(3.3.0)octyliden-7)-pentansäure. NMR: 7,4 - 7,9 (m); 5,2 (t); 5,0 (t); 4,4 (s).

15. 5E-(2ß-(2α-Hydroxy-3-butylthio-propylthio)-3α-hydroxy-bicyclo(3.3.0)octyliden-7)-pentansäure. NMR: 5,28 (t); 4,15 (Septett); 3,9 (m); 0,92 (t).

16. 5E-(2ß-(2α-Hydroxy-3-ethylthio-propylthio)-3α-hydroxy-bicyclo(3.3.0)octyliden-7)-pentansäure. NMR: 5,27 (t); 3,8 - 4,0 (m); 1,2 (t); 0,9 (t).

17. 5E-(2ß-(2α-Hydroxy-2ß-methyl-heptylthio)-3α-hydroxy-bicyclo(3.2.0)heptyliden-6)-pentansäure. IR: 3350, 2900, 1710, 740 $cm^{-1}$.

18. 5E-(2ß-(2α-Hydroxy-2-cyclohexyl-ethylthio)-3α-hydroxy-bicyclo(3.2.0)heptyliden-6)-pentansäure.

19. 5E-(2ß-(2α-Hydroxy-2-phenyl-ethylthio)-3α-hydroxy-bicyclo(3.2.0)heptyliden-6)-pentansäure. NMR: 7,2 - 7,4 (m); 5,1 (t); 4,8 (d); 4,2 (m).

20. 5E-(2ß-(2-Hydroxy-2-methyl-2-phenyl-ethylthio)-3α-hydroxy-bicyclo(3.2.0)heptyliden-6)-pentansäure. NMR: 7,2 - 7,6 (m); 5,15 (t); 4,12 (t); 1,63 (s).

21. 5E-(2ß-(2α-Hydroxy-2-o-chlorphenyl-ethylthio)-3α-hydroxy-bicyclo(3.2.0)heptyliden-6)-pentansäure. NMR: 7,63 (d); 7,2 - 7,4 (m); 5,22 (d); 5,12 (t); 4,22 (q).

22. 5E-(2ß-(2α-Hydroxy-2-m-chlorphenyl-ethylthio)-3α-hydroxy-bicyclo(3.2.0)heptyliden-6)-pentansäure. NMR: 7,45 (s); 7,3 (s); 5,22 (t); 4,7 - 4,9 (2d); 4,2 (q).

23. 5E-(2ß-(2α-Hydroxy-3-m-chlorphenoxy-propylthio)-3α-hydroxy-bicyclo(3.2.0)-heptyliden-6)-pentansäure. NMR: 4,9 - 5,2 (m); 3,9 - 4,3 (m); 2,7 - 3,1 (m).

24. 5-(2ß-(2-Hydroxy-2-methyl-heptylthio)-3α-hydroxy-6-oxa-bicylo(3.3.0)octyliden-7)-pentansäure.

25. 5-(2ß-(2α-Hydroxy-3-methyl-hept-5-inylthio)-3α-hydroxy-6-oxa-bicyclo(3.3.O)octyliden-7)-pentansäure.

26. 5-(2ß-(2α-Hydroxy-2-cyclohexyl-ethylthio)-3α-hydroxy-6-oxa-bicyclo(3.3.0)octyliden-7)-pentansäure.

27. 5-(2ß-(2α-Hydroxy-2-phenyl-ethylthio)-3α-hydroxy-6-oxa-bicyclo(3.3.0)octyliden-7)-pentansäure.

28. 5-(2ß-(2α-Hydroxy-3-m-chlorphenoxy-propylthio)-3α-hydroxy-6-oxa-bicyclo(3.3.0)octyliden-7)-pentansäure.

29. 5-(2ß-(2-Hydroxy-2-methyl-heptylthio)-3α-hydroxy-bicyclo(3.3.0)octyl-7)-pentansäure.

30. 5-(2ß-(2α-Hydroxy-2-cyclohexyl-ethylthio)-3α-hydroxy-bicyclo(3.3.0)octyl-7)-pentansäure.

31. 5-(2ß-(2α-Hydroxy-2-phenyl-ethylthio)-3α-hydroxy-bicyclo(3.3.0)octyl-7)-pentansäure. NMR: 7,2 - 7,4 (m); 4,8 (t); 4,2 (m).

32. 5-(2ß-(2α-Hydroxy-3-m-chlorphenoxy))-propylthio)-3α-hydroxy-bicyclo(3.3.0)octyl-7)-pentansäure. NMR: 6,8 - 7,3 (m); 4,1 - 4,2 (m); 3,9 - 4,05 (m).

33. 5-(2ß-(2-Hydroxy-2-methyl-heptylthio)-3α-hydroxy-bicyclo(3.2.0)heptyl-6)-pentansäure.

34. 5-(2ß-(2α-Hydroxy-2-cyclohexyl-ethylthio)-3α-hydroxy-bicyclo(3.2.0)heptyl-6)-pentansäure.

35. 5-(2ß-(2α-Hydroxy-2-phenyl-ethylthio)-3α-hydroxy-bicyclo(3.2.0)heptyl-6)-pentansäure.

36. 5-(2ß-(2α-Hydroxy-3-m-chlorphenoxy-propylthio)-3α-hydroxy-bicyclo(3.2.0)heptyl-6)-pentansäure.

37. 5-(2ß-(2-Hydroxy-2-methyl-heptylthio)-3α-hydroxy-6-oxa-bicyclo(3.3.0)octyl-7)-pentansäure, 2 Isomere; exo-Isomeres,NMR: 4,42 (q), 3,95 - 4,05 (m); endo-Isomeres, NMR: 3,85 (q), 2,5 - 3,0 (m).

38. 5-(2ß-(2α-Hydroxy-2-cyclohexyl-ethylthio)-3α-hydroxy-6-oxa-bicyclo(3.3.0)octyl-7)-pentansäure.

39. 5-(2ß-(2α-Hydroxy-2-phenyl-ethylthio)-3α-hydroxy-6-oxa-bicyclo(3.3.0)octyl-7)-pentansäure.NMR: 7,2 - 7,4 (m); 4,8 (t); 4,2 (m)

40. 5-(2ß-(2α-Hydroxy-3-m-chlorphenoxy-propylthio)-3α-hydroxy-6-oxa-bicyclo(3.3.0)-octyl-7)-pentansäure. NMR: 6,7 - 7,3 (m); 4,3 - 4,6 (q); 3,8 - 4,2 (m); 2,9 - 3,2 (2d).

Beispiel 41

Man löst 1 g 5E-(2ß-(2α-Hydroxy-2ß-methyl-heptylthio)-3α-acetoxy-bicyclo(3.3.0)octyliden-7)-pentansäuremethylester in 50 ml trockenem Methanol bei $0^{o}$, versetzt mit 0,1 g Natriummethylat, rührt 30 Minuten bei $0^{o}$ und anschließend 2 Stunden bei $20^{o}$. Man engt ein, nimmt den Rückstand in

Diethylether auf, filtriert, engt ein und erhält 5E-(2ß-(2α-Hydroxy-2ß-methyl-heptylthio)-3α-hydroxy-bicyclo(3.3.0)-octyliden-7)-pentansäuremethylester; NMR: 5,3 (t); 3,85 (q); 2,8 (q); 1,25 (s); 0,9(t).

Beispiele 42 bis 48

Analog Beispiel 41 sind aus den entsprechenden 3α-Acetoxy-Verbindungen der Formel IV erhältlich:

42. 5E-(2ß-(2α-Hydroxy-2-phenyl-ethylthio)-3α-hydroxy-bicyclo(3.3.0)octyliden-7)-pentansäuremethylester; NMR: 7,35 (s); 5,35 (t); 4,8 (2d); 3,7 - 4,1 (m); 2,7 - 3,2 (m); 1,2 - 2,5 (m).

43. 5E-(2ß-(2α-Hydroxy-3-m-chlorphenoxy-propylthio)-3α-hydroxy-bicyclo(3.3.0)octyliden-7)-pentansäuremethylester. NMR: 6,7 - 7,3 (m); 5,22 (t); 4,15 (q); 3,6 - 4,05 (m); 2,8 - 3,0 (m).

44. 5-(2ß-(2α-Hydroxy-2-phenyl-ethylthio)-3α-hydroxy-bicyclo(3.2.0)heptyliden-6)-pentan-säuremethylester.

45. 5-(2ß-(2α-Hydroxy-2-phenyl-ethylthio)-3α-hydroxy-6-oxa-bicyclo(3.3.0)octyliden-7)-pentansäuremethylester.

46. 5-(2ß-(2α-Hydroxy-2-phenyl-ethylthio)-3α-hydroxy-bicyclo(3.3.0)octyl-7)-pentansäure-methylester.

47. 5-(2ß-(2α-Hydroxy-2-phenyl-ethylthio)-3α-hydroxy-bicyclo(3.2.0.)heptyl-6)-pentansäure-methylester.

48. 5-(2ß-(2α-Hydroxy-2-phenyl-ethylthio)-3α-hydroxy-6-oxa-bicyclo(3.3.0)octyl-7)-pentansäuremethylester.

Beispiel 49

Man löst 3,6 g 7-(2ß-(2-Hydroxy-2-methyl-heptylthio)-3α,5α-dihydroxy-cyclopentyl)-hept-5Z-ensäuremethylester in 100 ml Ether und 13,5 g gesättigter Natriumbicarbonatlösung, versetzt tropfenweise mit einer Lösung von 10 g Jod und 7 g Kaliumjodid in 100 ml Wasser, rührt 5 Stunden und gibt dann gesättigte Natriumthiosulfatlösung bis zur Entfärbung hinzu. Die Etherphase wird gewaschen, getrocknet und eingeengt. Der Rückstand wird in 100 ml Benzol gelöst, mit 32 ml Diazabicyclononen versetzt und 8 Stunden gekocht. Nach üblicher Aufarbeitung (Toluol/Wasser, Zusatz von etwas Triethylamin) erhält man 5Z-(2ß-(2-Hydroxy-2-methyl-heptylthio)-3α-hydroxy-6-oxa-bicyclo(3.3.0)octyliden-7)-pentansäuremethylester. IR: 3350, 2900, 1725, 1700, 1440, 1050 $cm^{-1}$.

Beispiele 50 bis 65

Analog Beispiel 49 sind erhältlich:

50. 5Z-(2ß-(2-Hydroxy-octylthio)-3α-hydroxy-6-oxa-bicyclo(3.3.0)octyliden-7)-pentansäuremethylester.

51. 5Z-(2ß-(2-Hydroxy-2-methyl-octylthio)-3α-hydroxy-6-oxa-bicyclo(3.3.0)octyliden-7)-pentansäuremethylester.

52. 5Z-(2ß-(2-Hydroxy-hept-4-enylthio)-3α-hydroxy-6-oxa-bicyclo(3.3.0)octyliden-7)-pentansäuremethylester.

53. 5Z-(2ß-(2-Hydroxy-hept-4-inylthio)-3α-hydroxy-6-oxa-bicyclo(3.3.0)octyliden-7)-pentansäuremethylester.

54. 5Z-(2ß-(2α-Hydroxy-2-cyclohexyl-ethylthio)-3α-hydroxy-6-oxa-bicyclo(3.3.0)octyliden-7)-pentansäuremethylester.

55. 5Z-(2ß-(2α-Hydroxy-2-phenyl-ethylthio)-3α-hydroxy-6-oxa-bicyclo(3.3.0)octyliden-7)-pentansäuremethylester.

56. 5Z-(2ß-(2α-Hydroxy-3-phenyl-propylthio)-3α-hydroxy-6-oxa-bicyclo(3.3.0)octyliden-7)-pentansäuremethylester.

57. 5Z-(2ß-(2α-Hydroxy-2-o-chlorphenyl-ethylthio)-3α-hydroxy-6-oxa-bicyclo(3.3.0)-octyliden-7)-pentansäuremethylester.

58. 5Z-(2ß-(2α-Hydroxy-2-m-chlorphenyl-ethylthio)-3α-hydroxy-6-oxa-bicyclo(3.3.0)-octyliden-7)-pentansäuremethylester.

59. 5Z-(2ß-(2α-Hydroxy-2-p-chlorphenyl-ethylthio)-3α-hydroxy-6-oxa-bicyclo(3.3.0)-octyliden-7)-pentansäuremethylester.

60. 5Z-(2ß-(2α-Hydroxy-2-m-trifluormethylphenyl-ethylthio)-3α-hydroxy-6-oxa-bicyclo-(3.3.0)octyliden-7)-pentansäuremethylester.

61. 5Z-(2ß-(2α-Hydroxy-2-(2-naphthyl)-ethylthio)-3α-hydroxy-6-oxa-bicyclo(3.3.0)octyliden-7)-pentansäuremethylester.

62. 5Z-(2ß-(2α-Hydroxy-3-butylthio-propylthio)-3α-hydroxy-6-oxa-bicyclo(3.3.0)octyliden-7)-pentansäuremethylester.

63. 5Z-(2ß-(2α-Hydroxy-3-ethylthio-propylthio)-3α-hydroxy-6-oxa-bicyclo(3.3.0)octyliden-7)-pentansäuremethylester.

64. 5Z-(2ß-(2α-Hydroxy-3-phenoxy-propylthio)-3α-hydroxy-6-oxa-bicyclo(3.3.0)octyliden-7)-pentansäuremethylester.

65. 5Z-(2ß-(2α-Hydroxy-3-m-chlorphenoxy-propylthio)-3α-hydroxy-6-oxa-bicyclo(3.3.0)-octyliden-7)-pentansäuremethylester. NMR: 6,7 - 7,3 (m); 4,58 (t x 2); 3,9 - 4,25 (m); 3,63 (s); 1,0 - 3,0 (m).

Beispiel 66

Eine Lösung von 1,8 g Quecksilber-II-acetat in 10 ml THF und 15 ml Wasser wird tropfenweise mit einer Lösung von 1,05 g 7-(2ß-(2α-Hydroxy-3-m-chlorphenoxy-propylthio)-3α,5α-dihydroxy-cyclopentyl)-hept-5Z-ensäure in 5 ml THF versetzt. Man rührt noch 2 Std. nach und tropft dann eine Lösung von 0,5 g Natriumborhydrid in 15 ml Natronlauge zu. Nach weiteren 15 Minuten arbeitet man wie üblich auf (Ethylacetat/Salzsäure) und erhält 5-(2ß-(2α-Hydroxy-3-m-chlorphenoxy-propylthio)-3α-hydroxy-6-oxa-bicyclo(3.3.0)-octyl-7)-pentansäure. IR: 3300, 2900, 1705, 1590, 1240, 1040, 740 $cm^{-1}$; NMR: 6,7 - 7,3 (m); 4,3 - 4,6 (q); 3,8 - 4,2 (m); 2,9 - 3,2 (2d).

Beispiele 67 bis 74

Analog Beispiel 66 erhält man aus den entsprechenden Ausgangsverbindungen der Formel VI:

67. 5-(2ß-(2-Hydroxy-2-methyl-heptylthio)-3α-hydroxy-6-oxa-bicyclo(3.3.0)octyl-7)-pentansäure.

68. 5-(2ß-(2-Hydroxy-octylthio)-3α-hydroxy-6-oxa-bicyclo(3.3.0)octyl-7)-pentansäure.

69. 5-(2ß-(2α-Hydroxy-2-cyclohexyl-ethylthio)-3α-hydroxy-6-oxa-bicyclo(3.3.0)octyl-7)-pentansäure.

70. 5-(2ß-(2α-Hydroxy-2-phenyl-ethylthio)-3α-hydroxy-6-oxa-bicyclo(3.3.0)octyl-7)-pentansäure. NMR: 7,2 - 7,4 (m); 4,8 (t); 4,2 (m).

71. 5-(2ß-(2α-Hydroxy-3-phenyl-propylthio)-3α-hydroxy-6-oxa-bicyclo(3.3.0)octyl-7)-pentansäure.

72. 5-(2ß-(2α-Hydroxy-2-o-chlorphenyl-ethylthio)-3α-hydroxy-6-oxa-bicyclo(3.3.0)octyl-7)-pentansäure.

73. 5-(2ß-(2α-Hydroxy-3-ethylthio-propylthio)-3α-hydroxy-6-oxa-bicyclo(3.3.0)octyl-7)-pentansäure.

74. 5-(2ß-(2α-Hydroxy-3-phenoxy-propylthio)-3α-hydroxy-6-oxa-bicyclo(3.3.0)octyl-7)-pentansäure.

Beispiel 75

Zu einer Lösung von 723 g Carboxybutyltriphenylphosphoniumbromid und 548 g Kalium-tert.butylat in 1,5 l THF tropft man eine Lösung von 25 g 2ß-(2α-Hydroxy-2-phenyl-ethylthio)-3α-hydroxy-bicyclo(3.3.0)octan-7-on in 100 ml THF und rührt über Nacht bei $20^{o}$. Anschließend werden 150 ml Wasser zugegeben, der Ansatz wird eingeengt und mit Ethylacetat gewaschen. Man säuert mit Oxalsäure an und extrahiert mit Ether. Die Etherphase wird mit Natriumsulfat getrocknet und dann mit einem Überschuß etherischer Diazomethanlösung versetzt. Man läßt 30 Minuten stehen, arbeitet

wie üblich auf und rührt das Rohprodukt über Nacht bei 20° in einer Lösung von 20 ml 1 n NaOH und 80 ml THF. Nach üblicher Aufarbeitung (Oxalsäure/Wasser/Ether) erhält man 5E-(2ß-(2α-Hydroxy-2-phenyl-ethylthio)-3α-hydroxy-bicyclo-(3.3.0)octyliden-7)-pentansäure. NMR: 7,35 (s); 5,35 (t); 4,8 (2d); 3,7 - 4,1 (m); 2,7 - 3,2 (m); 1,2 - 2,5 (m).

Beispiele 76 bis 127

Analog Beispiel 75 erhält man aus den entsprechenden Verbindungen der Formel VII:

76. 5E-(2ß-(2α-Hydroxy-heptylthio)-3α-hydroxy-bicyclo(3.3.0)octyliden-7)-pentansäure. NMR: 5,26 (t); 3,9 (q); 2,85; 3,05 (2d); 0,85 (t).

77. 5Z-(2ß-(2α-Hydroxy-heptylthio)-3α-hydroxy-bicyclo(3.3.0)octyliden-7)-pentansäure. NMR: 5,15 (t); 3,5 - 4,0 (m); 0,9 (t).

78. 5E-(2ß-(2α-Hydroxy-2ß-methyl-heptylthio)-3α-hydroxy-bicyclo(3.3.0)octyliden-7)-pentansäure. NMR: 5,3 (t); 3,85 (q); 2,8 (q); 1,25 (s); 0,9 (t).

79. 5E-(2ß-(2ß-Hydroxy-2α-methyl-heptylthio)-3α-hydroxy-bicyclo(3.3.0)octyliden-7)-pentansäure. NMR: 5,3 (t); 3,85 (q); 2,6 - 3,0 (m); 1,22 (s); 0,9 (t);

80. 5E-(2ß-(2α-Hydroxy-octylthio)-3α-hydroxy-bicyclo-(3.3.0)octyliden-7)-pentansäure. NMR: 5,28 (t); 3,6 - 4,0 (m); 2,85; 3,0 (2d); 0,85 (t).

81. 5E-(2ß-(2ß-Hydroxy-octylthio)-3α-hydroxy-bicyclo-(3.3.0)octyliden-7)-pentansäure. NMR: 5,22 (t); 3,6 - 4,0 (m); 2,8 (d); 0,9 (t).

82. 5Z-(2ß-(2α-Hydroxy-octylthio)-3α-hydroxy-bicyclo (3.3.0)octyliden-7)-pentansäure. NMR: 5,18 (t); 3,9 (q); 2,4 - 3,0 (m).

83. 5E-(2ß-(2α-Hydroxy-2ß-methyl-octylthio)-3α-hydroxy-bicyclo(3.3.0)octyliden-7)-pentansäure. NMR: 5,3 (t); 3,7 - 4,2 (m); 2,7 - 2,9 (m); 1,23 (s); 0,9 (t).

84. 5Z-(2ß-(2α-Hydroxy-2ß-methyl-octylthio)-3α-hydroxy-bicyclo(3.3.0)octyliden-7)-pentansäure. NMR: 5,17 (t); 3,7 - 4,1 (m); 2,6 - 3,0 (m); 0,9 (t).

85. 5E-(2ß-(2ß-Hydroxy-2α-methyl-octylthio)-3α-hydroxy-bicyclo(3.3.0)octyliden-7)-pentansäure. NMR: 5,30 (t); 3,88 (q); 2,7 - 3,0 (m); 0,9 (t).

86. 5Z-(2ß-(2ß-Hydroxy-2α-methyl-octylthio)-3α-hydroxy-bicyclo(3.3.0)octyliden-7)-pentansäure. NMR: 5,20 (t); 3,7 - 4,1 (m); 2,5 - 3,1 (m); 0,9 (t).

87. 5-(2ß-(2-Hydroxy-2-methyl-nonylthio)-3α-hydroxy-bicyclo(3.3.0)octyliden-7)-pentansäure.

88. 5-(2ß-(2-Hydroxy-dodecylthio)-3α-hydroxy-bicyclo(3.3.0)octyliden-7)-pentansäure.

89. 5-(2ß-(2-Hydroxy-hept-4-enylthio)-3α-hydroxy-bicyclo(3.3.0)octyliden-7)-pentansäure.

90. 5-(2ß-(2-Hydroxy-oct-4-enylthio)-3α-hydroxy-bicyclo(3.3.0)octyliden-7)-pentansäure.

91. 5-(2ß-(2-Hydroxy-hept-4-inylthio)-3α-hydroxy-bicyclo(3.3.0)octyliden-7)-pentansäure.

92. 5E-(2ß-(2α-Hydroxy-3-methyl-hept-5-inylthio)-3α-hydroxy-bicyclo(3.3.0)octyliden-7)-pentansäure.

93. 5-(2ß-(2-Hydroxy-oct-4-inylthio)-3α-hydroxy-bicyclo(3.3.0)octyliden-7)-pentansäure.

94. 5E-(2ß-(2α-Hydroxy-2-cyclohexyl-ethylthio)-3α-hydroxy-bicyclo(3.3.0)octyliden-7)-pentansäure.
NMR: 5,3 (t); 3,85 (q); 3,5 (q); 2,8 (d).

95. 5E-(2ß-(2ß-Hydroxy-2-cyclohexyl-ethylthio)-3α-hydroxy-bicyclo(3.3.0)octyliden-7)-pentansäure.
NMR: 5,3 (t); 3,9 (q); 3,5 (q); 2,6 - 3,0 (m).

96. 5Z-(2ß-(2α-Hydroxy-2-cyclohexyl-ethylthio)-3α-hydroxy-bicyclo(3.3.0)octyliden-7)-pentansäure.
NMR: 5,22 (t); 3,9 (q); 3,5 (q); 2,6 - 3,0 (m).

97. 5Z-(2ß-(2ß-Hydroxy-2-cyclohexyl-ethylthio)-3α-hydroxy-bicyclo(3.3.0)octyliden-7)-pentansäure.
NMR: 5,22 (t); 3,95 (q); 3,55 (q); 2,85 (d).

98. 5E-(2ß-(2ß-Hydroxy-2-phenyl-ethylthio)-3α-hydroxy-bicyclo(3.3.0)octyliden-7)-pentansäure.
NMR: 5,25 (t); 4,85 (t); 3,9 (q); 2,9 - 3,0 (m).

99. 5Z-(2ß-(2α-Hydroxy-2-phenyl-ethylthio)-3α-hydroxy-bicyclo(3.3.0)octyliden-7)-pentansäure.
NMR: 7,35 (s); 5,25 (t); 4,8 (2d); 3,95 (q); 2,7 - 3,2 (m).

100. 5E-(2ß-(2-Hydroxy-2-methyl-2-phenyl-ethylthio)-3α-hydroxy-bicyclo(3.3.0)octyliden-7)-pentansäure.
NMR: 7,3 (s); 5,22 (t); 3,8 (t); 2,8 (s); 1,22 (s).

101. 5E-(2ß-(2α-Hydroxy-3-phenyl-propylthio)-3α-hydroxy-bicyclo(3.3.0)octyliden-7)-pentansäure. NMR: 7,3 (s); 5,2 (t); 3,8 - 4,2 (m).

102. 5E-(2ß-(2ß-Hydroxy-3-phenyl-propylthio)-3α-hydroxy-bicyclo(3.3.0)octyliden-7)-pentansäure. NMR: 7,2 - 7,4 (m); 5,2 (t); 3,8 - 4,2 (m).

103. 5E-(2ß-(2α-Hydroxy-2-o-chlorphenyl-ethylthio)-3α-hydroxy-bicyclo(3.3.0)octyliden-7)-pentansäure. NMR: 7,45 - 7,6 (m); 7,1 - 7,3 (m); 5,20 (t); 5,10 (2d); 3,88 (q).

104. 5E-(2ß-(2ß-Hydroxy-2-o-chlorphenyl-ethylthio)-3α-hydroxy-bicyclo(3.3.0)octyliden-7)-pentansäure. NMR: 7,2 - 7,8 (m); 5,28 (d); 5,25 (t); 2,8 - 3,1 (m).

105. 5E-(2ß-(2α-Hydroxy-2-m-chlorphenyl-ethylthio)-3α-hydroxy-bicyclo(3.3.0)octyliden-7)-pentansäure. NMR: 7,35 (s); 7,22 (m); 5,28 (t); 4,7 - 4,8 (m); 3,9 (q).

106. 5E-(2ß-(2α-Hydroxy-2-p-chlorphenyl-ethylthio)-3α-hydroxy-bicyclo(3.3.0)octyliden-7)-pentansäure. NMR: 7,3 (d); 4,25 (t); 2,6 - 3,0 (m).

107. 5E-(2ß-(2ß-Hydroxy-2-p-chlorphenyl-ethylthio)-3α-hydroxy-bicyclo(3.3.0)octyliden-7)-pentansäure. NMR: 7,3 (s); 5,1 (t); 4,9 (t); 2,95 (d).

108. 5E-(2ß-(2α-Hydroxy-2-o-methoxyphenyl-ethylthio)-3α-hydroxy-bicyclo(3.3.0)octyliden-7)-pentansäure.

109. 5E-(2ß-(2α-Hydroxy-2-m-trifluormethylphenyl-ethylthio)-3α-hydroxy-bicyclo(3.3.0)-octyliden-7)-pentansäure. NMR: 7,3 - 7,6 (m); 5,3 (t); 4,9 (t); 3,9 (q); 2,9 (2d).

110. 5E-(2ß-(2α-Hydroxy-2-(2-pyridyl-ethylthio)-3α-hydroxy-bicyclo(3.3.0)octyliden-7)-pentansäure.

111. 5E-(2ß-(2α-Hydroxy-2-(2-naphthyl)-ethylthio)-3α-hydroxy-bicyclo(3.3.0)octyliden-7)-pentansäure. NMR: 7,4 - 7,9 (m); 5,2 (t); 5,0 (t); 4,4 (s).

112. 5E-(2ß-(2α-Hydroxy-2-(2-thienyl)-ethylthio-)3α-hydroxy-bicyclo(3.3.0)octyliden-7)-pentansäure. NMR: 7,25 (t); 7,0 (t); 5,32 (t); 5,05 - 5,2 (m); 3,93 (q); 3,15 - 3,27 (m); 2,85 - 3,0 (m).

113. 5E-(2ß-(2ß-Hydroxy-2-(2-thienyl)-ethylthio)-3α-hydroxy-bicyclo(3.3.0)octyliden-7)-pentansäure. NMR: 7,26 (t), 7,0 (t), 5,3 (t), 5,17 (q), 3,9 (q).

114. 5E-(2ß-(2α-Hydroxy-2-(3-thienyl)-ethylthio)-3α-hydroxy-bicyclo(3.3.0)octyliden-7)-pentansäure.

115. 5E-(2ß-(2α-Hydroxy-3-ethoxy-propylthio)-3α-hydroxy-bicyclo(3.3.0)octyliden-7)-pentansäure.

116. 5E-(2ß-(2α-Hydroxy-3-butoxy-propylthio)-3α-hydroxy-bicyclo(3.3.0)octyliden-7)-pentansäure.

117. 5E-(2ß-(2α-Hydroxy-3-butylthio-propylthio)-3α-hydroxy-bicyclo(3.3.0)octyliden-7)-pentansäure. NMR: 5,28 (t); 4,15 (Septett); 3,9 (m); 0,92 (t).

118. 5-(2ß-(2ß-Hydroxy-3-butylthio-propylthio)-3α-hydroxy-bicyclo(3.3.0)octyliden-7-pentansäure.
NMR: 5,26 (t); 3,8 - 4,3 (m).

119. 5Z-(2ß-(2α-Hydroxy-3-butylthio-propylthio)-3α-hydroxy-bicyclo(3.3.0)octyliden-7)-pentansäure.
NMR: 5,18 (t); 3,8 - 4,3 (m); 0,9 (t).

120. 5E-(2ß-(2α-Hydroxy-3-ethylthio-propylthio)-3α-hydroxy-bicyclo(3.3.0)octyliden-7)-pentansäure.
NMR: 5,27 (t); 3,8 - 4,0 (m); 1,2 (t); 0,9 (t).

121. 5E-(2ß-(2ß-Hydroxy-3-ethylthio-propylthio)-3α-hydroxy-bicyclo(3.3.0)octyliden-7)-pentansäure.
NMR: 5,26 (t), 3,8 - 4,0 (m); 0,9 (t).

122. 5E-(2ß-(2α-Hydroxy-3-phenoxy-propylthio)-3α-hydroxy-bicyclo(3.3.0)octyliden-7)-pentansäure.

123. 5E-(2ß-(2α-Hydroxy-3-m-chlorphenoxy-propylthio)-3α-hydroxy-bicyclo(3.3.0)-octyliden-7)-pentansäure.
NMR: 7,15 - 7,25 (m); 5,28 (t); 4,15 (q); 3,15 - 3,25; 2,66 - 2,78 (m).

124. 5Z-(2ß-(2α-Hydroxy-3-m-chlorphenoxy-propylthio)-3α-hydroxy-bicyclo(3.3.0)octyliden-7)-pentansäure.
NMR: 6, 8 - 7,25 (m); 5,28 (t); 4,12 (q); 3,05 - 3,15; 2,66 - 2,78 (m).

125. 5E-(2ß-(2ß-Hydroxy-3-m-chlorphenoxy-propylthio)-3α-hydroxy-bicyclo(3.3.0)octyliden-7)-pentansäure.
NMR: 5,22 (t); 3,8 - 4,2 (m); 2,85 - 3,0 (m).

126. 5Z-(2ß-(2ß-Hydroxy-3-m-chorphenoxy-propylthio)-3α-hydroxy-bicyclo(3.3.0)octyliden-7)-pentansäure.
NMR: 6,7 - 7,3 (m); 5,22 (t); 4,15 (q); 3,6 - 4,05 (m); 2,8 - 3,0 (m).

127. 5E-(2ß-(2α-Hydroxy-3-m-trifluormethylphenoxy-propylthio)-3α-hydroxy-bicyclo(3.3.0)-octyliden-7)-pentansäure.

Beispiel 128

Eine Lösung von 16,8 g Kalium-tert.butylat in 100 ml THF wird bei 20° portionsweise mit 20 g Carboxybutyltriphenylphosphoniumbromid versetzt. Man rührt 30 Minuten nach und tropft eine Lösung von 8,58 g 2ß-(2-Hydroxy-2-methyl-heptylthio)-3α-hydroxy-bicyclo(3.2.0)heptan-6-on in 40 ml THF zu. Nach weiteren 30 Minuten gibt man Wasser hinzu, arbeitet wie üblich auf (Oxalsäure/Wasser/Ether) und erhält 5-(2ß-(2-Hydroxy-2-methyl-heptylthio)-3α-hydroxy-bicyclo(3.2.0)heptyliden-6)-pentansäure. IR: 3350, 2900, 1710, 740 $cm^{-1}$.

Beispiele 129 bis 145

Analog Beispiel 128 sind aus den entsprechenden Verbindungen der Formel VII erhältlich:

129. 5-(2ß-(2-Hydroxy-heptylthio)-3α-hydroxy-bicyclo(3.2.0)heptyliden-6)-pentansäure.

130. 5-(2ß-(2-Hydroxy-octylthio)-3α-hydroxy-bicyclo(3.2.0)heptyliden-6)-pentansäure.

131. 5E-(2ß-(2α-Hydroxy-3-methyl-hept-5-inylthio)-3α-hydroxy-bicyclo(3.2.0)heptyliden-6)-pentansäure.

132. 5E-(2ß-(2α-Hydroxy-2-cyclohexyl-ethylthio)-3α-hydroxy-bicyclo(3.2.0)heptyliden-6)-pentansäure.

133. 5E-(2ß-(2α-Hydroxy-2-phenyl-ethylthio)-3α-hydroxy-bicyclo(3.2.0)heptyliden-6)-pentansäure. NMR: 7,2 - 7,4 (m); 5,1 (t); 4,8 (d); 4,2 (m).

134. 5E-(2ß-(2ß-Hydroxy-2-phenyl-ethylthio)-3α-hydroxy-bicyclo(3.2.0)heptyliden-6)-pentansäure. NMR: 7,2 - 7,4 (m); 5,1 (t); 4,8 (t); 4,2 (m); 2,9 (m).

135. 5-(2ß-(2-Hydroxy-2-methyl-2-phenyl-ethylthio)-3α-hydroxy-bicyclo(3.2.0)heptyliden-6)-pentansäure. NMR: 7,2 - 7,6 (m); 5,15 (t); 4,12 (t); 1,63 (s).

136. 5-(2ß-(2α-Hydroxy-2-o-chlorphenyl-ethylthio)-3α-hydroxy-bicyclo(3.2.0)heptyliden-6)-pentansäure. NMR: 7,63 (d); 7,2 - 7,4 (m), 5,22 (d); 5,12 (t); 4,22 (q).

137. 5-(2ß-(2ß-Hydroxy-2-o-chlorphenyl-ethylthio)-3α-hydroxy-bicyclo(3.2.0)heptyliden-6)-pentansäure. NMR: 7,6 (d); 7,2 - 7,4 (m); 5,2 - 5,25 (m); 5,12 (t); 4,22 (q).

138. 5-(2ß-(2α-Hydroxy-2-m-chlorphenyl-ethylthio)-3α-hydroxy-bicyclo(3.2.0)heptyliden-6)-pentansäure. NMR: 7,45 (s); 7,3 (s); 5,22 (t); 4,7 - 4,9 (2d); 4,2 (q).

139. 5-(2ß-(2ß-Hydroxy-2-m-chlorphenyl-ethylthio)-3α-hydroxy-bicyclo(3.2.0)heptyliden-6)-pentansäure. NMR: 7,43 (s); 7,3 (s); 5,12 (t); 4,7 - 4,9 (2d).

140. 5-(2ß-(2α-Hydroxy-3-phenyl-propylthio)-3α-hydroxy-bicyclo(3.2.0)heptyliden-6)-pentansäure. NMR: 7,2 - 7,4 (m); 5,1 (t); 4,8 (d); 4,2 (m).

141. 5-(2ß-(2α-Hydroxy-2-p-chlorphenyl-ethylthio)-3α-hydroxy-bicyclo(3.2.0)heptyliden-6)-pentansäure.

142. 5-(2ß-(2α-Hydroxy-3-ethylthio-propylthio)-3α-hydroxy-bicyclo(3.2.0)heptyliden-6)-pentansäure.

143. 5-(2ß-(2α-Hydroxy-3-phenoxy-propylthio)-3α-hydroxy-bicyclo(3.2.0)heptyliden-6)-pentansäure.

144. 5-(2ß-(2α-Hydroxy-3-m-chlorphenoxy-propylthio)-3α-hydroxy-bicyclo(3.2.0)heptyliden-6)-pentansäure. NMR: 4,9 - 5,2 (m); 3,9 - 4,3 (m); 2,7 - 3,1 (m).

145. 5-(2ß-(2ß-Hydroxy-3-m-chlorphenoxy-propylthio)-3α-hydroxy-bicyclo(3.2.0)heptyliden-6)-pentansäure; NMR: 4,9 - 5,2 (m); 3,9 - 4,3 (m); 2,85 (d).

Beispiel 146

1,5 g 5E-(2ß-(2α-Hydroxy-2ß-methyl-heptylthio)-3α-hydroxy-bicyclo(3.3.0)octyliden-7)-pentansäure werden in 50 ml Ethylacetat gelöst, mit 0,5 g Pd/C versetzt und 3 Stunden bei 20$^{o}$ und 1 bar Wasserstoff hydriert. Man filtriert, engt ein und erhält 5-(2ß-(2α-Hydroxy-2ß-methyl-heptylthio)-3α-hydroxy-bicyclo(3.3.0)octyl-7)-pentansäure. NMR: 3,6 - 4,3 (m); 3,6 (s); 1,2 (s); 0,9 (t).

Beispiele 147 bis 155

Analog Beispiel 146 erhält man durch Hydrierung:

147. 5-(2ß-(2-Hydroxy-octylthio)-3α-hydroxy-bicyclo(3.3.0)octyl-7)-pentansäure.

148. 5-(2ß-(2α-Hydroxy-2-cyclohexyl-ethylthio)-3α-hydroxy-bicyclo(3.3.0)octyl-7)-pentansäure.

149. 5-(2ß-(2α-Hydroxy-2-phenyl-ethylthio)-3α-hydroxy-bicyclo(3.3.0)octyl-7)-pentansäure.

150. 5-(2ß-(2α-Hydroxy-3-phenyl-propylthio)-3α-hydroxy-bicyclo(3.3.0)octyl-7)-pentansäure.

151. 5-(2ß-(2α-Hydroxy-2-o-chlorphenyl-ethylthio)-3α-hydroxy-bicyclo(3.3.0)octyl-7)-pentansäure.

152. 5-(2ß-(2α-Hydroxy-3-ethylthio-propylthio)-3α-hydroxy-bicyclo(3.3.0)octyl-7)-pentansäure.

153. 5-(2ß-(2α-Hydroxy-3-phenoxy-propylthio)-3α-hydroxy-bicyclo(3.3.0)octyl-7)-pentansäure.

154. 5-(2ß-(2α-Hydroxy-3-m-chlorphenoxy-propylthio)-3α-hydroxy-bicyclo(3.3.0)octyl-7)-pentansäure.
NMR: 6,8 - 7,3 (m); 4,1 - 4,2 (m); 3,9 - 4.05 (m).

155. 5-(2ß-(2ß-Hydroxy-3-m-chlorphenoxy-propylthio)-3α-hydroxy-bicyclo(3.3.0)octyl-7)-pentansäure.
NMR: 7,18 - 7,35; 6,8 - 7,0 (m); 4,0 (t).

Beispiel 156

Eine Lösung von 1 g 5E-(2ß-(2α-Hydroxy-2ß-methyl-heptylthio)-3α-hydroxy-bicyclo(3.3.0)octyliden-7)-pentansäure in 30 ml Ether und 10 ml Methanol wird mit etherischer Diazomethanlösung versetzt. Man läßt 30 Minuten stehen, gibt Essigsäure zu, engt ein und erhält 5E-(2ß-(2α-Hydroxy-2ß-methyl-heptylthio)-3α-hydroxy-bicyclo(3.3.0)octyliden-7)-pentansäuremethylester.

Beispiele 157 bis 186

Analog Beispiel 156 sind aus den entsprechenden Säuren der Formel I mit Diazomethan erhältlich:

157. 5E-(2ß-(2α-Hydroxy-2-cyclohexyl-ethylthio)-3α-hydroxy-bicyclo(3.3.0)octyliden-7)-pentansäuremethylester.

158. 5E-(2ß-(2α-Hydroxy-2-phenyl-ethylthio-3α-hydroxy-bicyclo(3.3.0)octyliden-7)-pentansäuremethylester. NMR: 7,35 (s); 5,35 (t); 4,8 (2d); 3,7 - 4,1 (m); 2,7 - 3,2 (m); 1,2 - 2,5 (m).

159. 5E-(2ß-(2ß-Hydroxy-2-phenyl-ethylthio)-3α-hydroxy-bicyclo(3.3.0)octyliden-7)-pentansäuremethylester; NMR: 5,28 (t); 4,9 (t); 3,7 (s); 2,8 - 3,0 (m).

160. 5E-(2ß-(2α-Hydroxy-3-m-chlorphenoxy-propylthio)-3α-hydroxy-bicyclo(3.3.0)octyliden-7)-pentansäuremethylester. NMR: 6,7 - 7,3 (m); 5,22 (t); 4,15 (q); 3,6 - 4,05 (m); 2,8 - 3,0 (m).

161. 5E-(2ß-(2ß-Hydroxy-3-m-chlorphenoxy-propylthio)-3α-hydroxy-bicyclo(3.3.0)octyliden-7)-pentansäuremethylester. NMR: 5,23 (t); 4,0 (t); 3,66 (s).

162. 5E-(2ß-(2α-Hydroxy-3-phenyl-propylthio)-3α-hydroxy-bicyclo(3.3.0)octyliden-7)-pentansäuremethylester. NMR: 7,3 (s); 5,2 (t); 3,8 - 4,2 (m); 3,7 (s).

163. 5E-(2ß-(2ß-Hydroxy-3-phenyl-propylthio)-3α-hydroxy-bicyclo(3.3.0)octyliden-7)-pentansäuremethylester. NMR: 7,2 - 7,4 (m); 5,2 (t); 3,8 - 4,2 (m); 3,7 (s).

164. 5E-(2ß-(2α-Hydroxy-3-ethylthio-propylthio)-3α-hydroxy-bicyclo(3.3.0)octyliden-7)-pentansäuremethylester; NMR: 5,27 (t); 3,8 - 4,0 (m); 3,7 (s); 1,2 (t); 0,9 (t).

165. 5E-(2ß-(2ß-Hydroxy-3-ethylthio-propylthio)-3α-hydroxy-bicyclo(3.3.0)octyliden-7)-pentansäuremethylester; NMR: 5,26 (t); 3,8 - 4,0 (m); 3,65 (s); 0,9 (t).

166. 5Z-(2ß-(2α-Hydroxy-2ß-methyl-heptylthio)-3α-hydroxy-6-oxa-bicyclo(3.3.0)octyliden-7)-pentansäuremethylester. IR: 3350, 2900, 1725, 1700, 1440, 1050 $cm^{-1}$.

167. 5-(2ß-(2α-Hydroxy-2-cyclohexyl-ethylthio)-3α-hydroxy-6-oxa-bicyclo(3.3.0)octyliden-7)-pentansäuremethylester.

168. 5-(2ß-(2α-Hydroxy-2-phenyl-ethylthio)-3α-hydroxy-6-oxa-bicyclo(3.3.0)octyliden-7)-pentansäuremethylester.

169. 5Z-(2ß-(2α-Hydroxy-3-m-chlorphenoxy-propylthio)-3α-hydroxy-6-oxa-bicyclo(3.3.0)-octyliden-7)-pentansäuremethylester. NMR: 6,7 - 7,3 (m), 4,58 (t x 2); 3,9 - 4,25 (m); 3,63 (s); 1,0 - 3,0 (m).

170. 5Z(2ß-(2ß-Hydroxy-3-m-chlorphenoxy-propylthio)-3α-hydroxy-bicyclo(3.3.0)octyliden-7)-pentansäuremethylester.

171. 5-(2ß-(2α-Hydroxy-3-phenyl-propylthio)-3α-hydroxy-6-oxa-bicyclo(3.3.0)octyliden-7)-pentansäuremethylester.

172. 5-(2ß-(2-Hydroxy-2-methyl-heptylthio)-3α-hydroxy-bicyclo(3.2.0)heptyliden-6)-pentansäuremethylester.

173. 5-(2ß-(2α-Hydroxy-2-cyclohexyl-ethylthio)-3α-hydroxy-bicyclo(3.2.0)heptyliden-6)-pentansäuremethylester.

174. 5-(2ß-(2α-Hydroxy-2-phenyl-ethylthio)-3α-hydroxy-bicyclo(3.2.0)heptyliden-6)-pentansäuremethylester.

175. 5-(2ß-(2α-Hydroxy-3-m-chlorphenoxy-propylthio)-3α-hydroxy-bicyclo(3.2.0)heptyliden-6)-pentansäuremethylester.

176. 5-(2ß-(2α-Hydroxy-3-phenyl-propylthio)-3α-hydroxy-bicyclo(3.2.0)heptyliden-6)-pentansäuremethylester.

177. 5-(2ß-(2-Hydroxy-2-methyl-heptylthio)-3α-hydroxy-6-oxa-bicyclo(3.3.0)octyl-7)-pentansäuremethylester.

178. 5-(2ß-(2α-Hydroxy-2-cyclohexyl-ethylthio)-3α-hydroxy-6-oxa-bicyclo(3.3.0)octyl-7)-pentansäuremethylester.

179. 5-(2ß-(2α-Hydroxy-2-phenyl-ethylthio)-3α-hydroxy-6-oxa-bicyclo(3.3.0)octyl-7)-pentansäuremethylester.

180. 5-(2ß-(2α-Hydroxy-3-m-chlorphenoxy-propylthio)-3α-hydroxy-6-oxa-bicyclo(3.3.0)octyl-7)-pentansäuremethylester.

181. 5-(2ß-(2α-Hydroxy-3-phenyl-propylthio)-3α-hydroxy-6-oxa-bicyclo(3.3.0)octyl-7)-pentansäuremethylester.

Beispiel 182

Eine Lösung von 1 g 5-(2ß-(2α-Hydroxy-2-phenyl-ethylthio)-3α-hydroxy-bicyclo(3.3.0)octyliden-7)-pentansäure in 10 ml Ethanol wird bei 0° mit HCl gesättigt. Man läßt 12 Stunden bei 20° stehen, filtriert, arbeitet wie üblich auf (Dichlormethan/gesättigte Natriumbicarbonat-Lösung) und erhält 5-(2ß-(2α-Hydroxy-2-phenyl-ethylthio)-3α-hydroxy-bicyclo(3.3.0)octyliden-7)-pentansäureethylester.

Beispiele 183 bis 186

Analog Beispiel 182 sind durch Umsetzen mit n-Propanol, Isopropanol, n-Butanol oder Isobutanol erhältlich:

183. 5-(2ß-(2α-Hydroxy-2-phenyl-ethylthio)-3α-hydroxy-bicyclo(3.3.0)octyliden-7)-pentansäure-n-propylester.

184. 5-(2ß-(2α-Hydroxy-2-phenyl-ethylthio)-3α-hydroxy-bicyclo(3.3.0)octyliden-7)-pentansäure-i-propylester.

185. 5-(2ß-(2α-Hydroxy-2-phenyl-ethylthio)-3α-hydroxy-bicyclo(3.3.0)octyliden-7)-pentansäure-n-butylester.

186. 5-(2ß-(2α-Hydroxy-2-phenyl-ethylthio)-3α-hydroxy-bicyclo(3.3.0)octyliden-7)-pentansäure-i-butylester.

Beispiel 187

Eine Lösung von 8 g 5E-(2ß-(2α-Hydroxy-2-cyclohexyl-ethylthio)-3α-hydroxy-bicyclo(3.3.0)octyliden-7)-pentansäure, 7,7 g p-Benzamidophenol, 5,5 g Dicyclohexylcarbodiimid und 0,75 g 4-Dimethylaminopyridin in 200 ml Dichlormethan und 50 ml DMF wird 1 Stunde bei $0^{o}$ und anschließend 3 Stunden bei $20^{o}$ gerührt. Man filtriert, arbeitet wie üblich auf und erhält 5E-(2ß-(2α-Hydroxy-2-cyclohexyl-ethylthio)-3α-hydroxy-bicyclo(3.3.0)octyliden-7)-pentansäure-p-benzamidophenylester. F. $87^{o}$.

Beispiele 188 bis 194

Analog Beispiel 187 sind die folgenden Ester der Formel I erhältlich:

188. 5E-(2ß-(2α-Hydroxy-2-phenyl-ethylthio)-3α-hydroxy-bicyclo(3.3.0)octyliden-7)-pentansäure-p-benzamidophenylester. NMR: 7,05 - 8,0 (m); 5,35 (t); 4,75 - 4,85 (m); 3.95 (q); 2,7 - 3,15 (m).

189. 5E-(2ß-(2α-Hydroxy-2-phenyl-ethylthio)-3α-hydroxy-bicyclo(3.3.0)octyliden-7)-pentansäure-p-acetamidophenylester.

190. 5E-(2ß-(2α-Hydroxy-2-phenyl-ethylthio)-3α-hydroxy-bicyclo(3.3.0)octyliden-7)-pentansäure-p-ureidophenylester.

191. 5E-(2ß-(2α-Hydroxy-2-phenyl-ethylthio)-3α-hydroxy-bicyclo(3.3.0)octyliden-7)-pentansäurephenylester.

192. 5E-(2ß-(2α-Hydroxy-2-phenyl-ethylthio)-3α-hydroxy-bicyclo(3.3.0)octyliden-7)-pentansäure-p-tolylester.

193. 5E-(2ß-(2α-Hydroxy-2-phenyl-ethylthio)-3α-hydroxy-bicyclo(3.3.0)octyliden-7)-pentansäure-1-naphthylester.

194. 5E-(2ß-(2α-Hydroxy-2-phenyl-ethylthio)-3α-hydroxy-bicyclo(3.3.0)octyliden-7)-pentansäure-p-biphenylylester.

Beispiel 195

Eine Lösung von 1,85 g 5E-(2ß-(2α-Hydroxy-heptylthio)-3α-hydroxy-bicyclo(3.3.0)octyliden-7)-pentansäure in 30 ml absolutem Methanol wird mit 50 ml 0,1 n Natronlauge versetzt. Die wäßrige Lösung wird konzentriert und gefriergetrocknet. Man erhält das Natriumsalz der 5E-(2ß-(2α-Hydroxy-heptylthio)-3α-hydroxy-bicyclo(3.3.0)octyliden-7)-pentansäure.

Die nachstehenden Beispiele betreffen Mischungen von Verbindungen der Formel I mit in der Pharmazie üblichen Träger- oder Hilfsstoffen, welche vor allem als Arzneimittel verwendet werden können:

Beispiel A: Tabletten

Ein Gemisch, bestehend aus 3 g 5E-(2ß-(2α-Hydroxy-2-phenyl-ethylthio)-3α-hydroxy-bicyclo(3.3.0)octyliden-7)-pentansäure, 50 g Lactose, 16 g Maisstärke, 2 g Cellulosepulver und 2 g Magnesiumstearat, wird in üblicher Weise zu Tabletten gepreßt, derart, daß jede Tablette 1 mg des Wirkstoffes enthält.

Beispiel B: Dragees

Analog Beispiel A werden Tabletten gepreßt, die anschließend in üblicher Weise mit einem Überzug, bestehend aus Zucker, Maisstärke, Talk und Tragant, überzogen werden.

Beispiel C: Injektionslösung

0,1 g 5-(2ß-(2α-Hydroxy-2ß-methyl-heptylthio)-3α-hydroxy-bicyclo(3.3.0)octyliden-7)-pentansäure werden in einem Gemisch aus 4 l destilliertem Wasser, 0,5 l Ethanol und 0,5 l Propylenglykol gelöst, die Lösung wird steril filtriert. Die erhaltene Injektionslösung wird je nach Bedarf in Ampullen enthaltend 2,5 ml, 5 ml oder 10 ml der Injektionslösung gefüllt. Jede Ampulle enthält 0,05, 0,1 oder 0,2 mg Wirkstoff.

Analog sind Tabletten, Dragees und Injektionslösungen erhältlich, die einen oder mehrere der übrigen Wirkstoffe der Formel I enthalten.

Merck Patent Gesellschaft
mit beschränkter Haftung
D a r m s t a d t

Patentansprüche:

1. 13-Thia-Prostacycline der allgemeinen Formel I

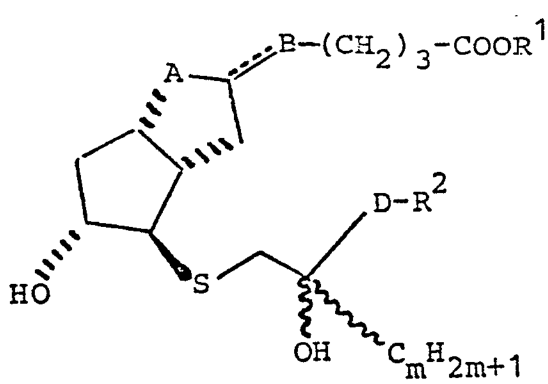

(I)

worin

A -O-, $-CH_2-$ oder eine Bindung,

B $-CH_2-$ oder =CH-,

D eine Bindung, Alkylen mit 1 - 3 C-Atomen, cis-Alkenylen mit 2 - 5 C-Atomen oder Alkinylen mit 2 - 5 C-Atomen,

m 0, 1, 2 oder 3

$R^1$ H, Alkyl mit 1 - 4 C-Atomen, Aryl mit 6 - 12 C-Atomen oder $-C_6H_4-NH-CO-R^3$,

$R^2$ Alkyl mit 1 - 7 C-Atomen, durch Halogen substituiertes Alkyl mit 1 - 7 C-Atomen, Cycloalkyl mit 5 - 6 C-Atomen, durch Alkyl mit 1 - 4 C-Atomen substituiertes Cycloalkyl mit 5 - 6 C-Atomen, Phenyl, durch F, Cl, Br, Alkyl mit 1 - 4 C-Atomen, OH, $OCH_3$ oder $CF_3$ substituiertes Phenyl, Pyridyl, Naphthyl, Thienyl, oder, falls D Alkylen mit 1 - 3 C-Atomen bedeutet, auch Alkoxy mit 1 - 4 C-Atomen, Alkylthio mit 1 - 4 C-Atomen, Phenoxy oder durch F, Cl, Br, Alkyl mit 1 - 4 C-Atomen, OH, $OCH_3$ oder $CF_3$ substituiertes Phenoxy,

$R^3$ $NH_2$, $CH_3$ oder Phenyl

bedeuten und

 anzeigt, daß diese Bindungen α- oder ß-ständig sein können, wobei eine der Bindungen α- und die andere ß-ständig ist,

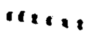 anzeigt, daß diese Bindung α-ständig ist,

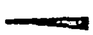 anzeigt, daß diese Bindung ß-ständig ist,

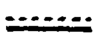 anzeigt, daß diese Bindung je nach Bedeutung von B eine Einfach- oder Doppelbindung ist, wobei die Einfachbindung α- oder ß-ständig und die Doppelbindung in der E- oder Z-Konfiguration sein kann,

sowie, falls $R^1$ = H ist, deren physiologisch unbedenkliche Metall- und Ammoniumsalze.

2. Verfahren zur Herstellung von 13-Thia-Prostacyclinen der allgemeinen Formel I nach Anspruch 1

sowie, falls $R^1$ = H ist, von deren physiologisch unbedenklichen Metall- und Ammoniumsalzen, dadurch gekennzeichnet, daß man

a) eine Verbindung der allgemeinen Formel II

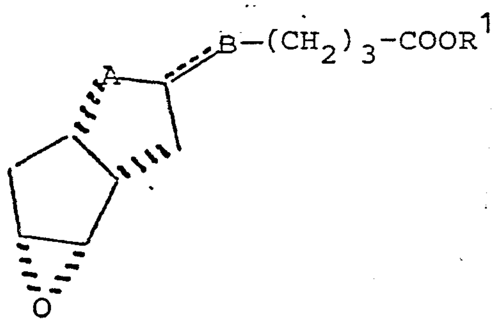

(II)

worin

A, B und $R^1$ die oben angegebenen Bedeutungen haben,

mit einer Verbindung der allgemeinen Formel III

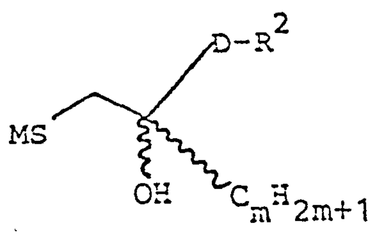

(III)

worin

M H, ein Äquivalent eines Alkali- oder Erdalkalimetallatoms oder ein gegebenenfalls substutiertes Ammoniumion bedeutet, und

D, m und $R^2$ die oben angegebenen Bedeutungen haben,

umsetzt,

oder daß man

b) in einer Verbindung der allgemeinen Formel IV

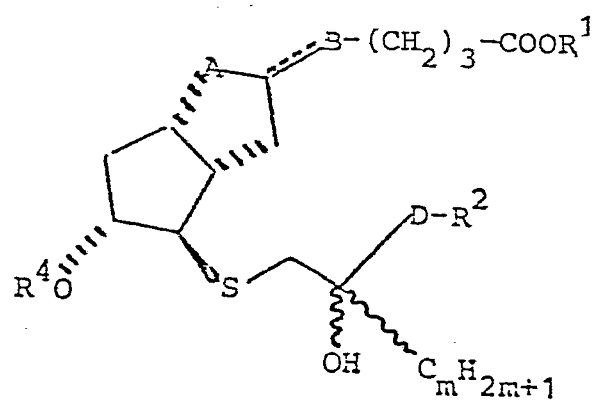

(IV)

worin

$R^4$ eine solvolytisch leicht abspaltbare Schutzgruppe bedeutet, und

A, B, D, m und $R^1$ bis $R^3$ die oben angegebenen Bedeutungen haben,

die Schutzgruppe unter neutralen oder alkalischen Bedingungen abspaltet,

oder daß man

c) aus einer Verbindung der allgemeinen Formel V

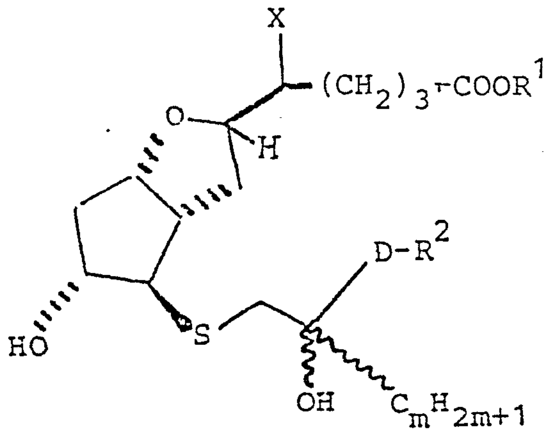

(V)

worin

X Chlor, Brom oder Jod bedeutet, und
D, m und $R^1$ bis $R^3$ die oben angegebenen Bedeutungen haben,

HX abspaltet,

wobei man eine Verbindung der allgemeinen Formel I erhält, in der A -O- und B =CH- bedeutet,

oder daß man

d) eine Verbindung der allgemeinen Formel VI

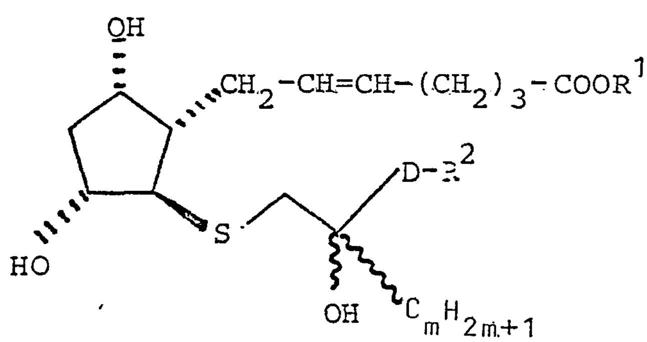

(VI)

worin

D, m und $R^1$ bis $R^3$ die oben angegebenen Bedeutungen haben,

mit einem elektrophilen Reagenz umsetzt und anschließend mit einem Reduktionsmittel behandelt,

wobei man eine Verbindung der allgemeinen Formel I erhält, in der A -O- und B $-CH_2-$ bedeutet,

oder daß man

e) eine Verbindung der allgemeinen Formel VII

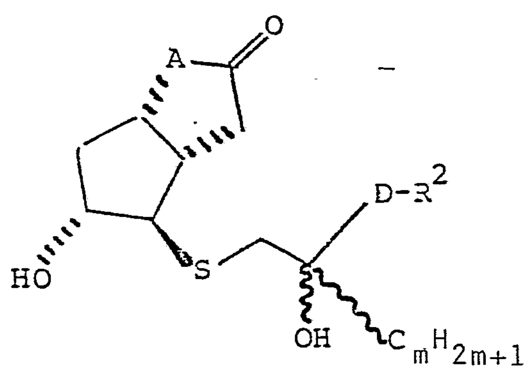

(VII)

worin

A $-CH_2-$ oder eine Bindung bedeutet und
D, m und $R^2$ die oben angegebenen Bedeutungen haben,

mit einem Ylid der allgemeinen Formel VIII

$$(C_6H_5)_3P=CH-(CH_2)_3-COO^{(-)}\ Q^{(+)} \qquad \text{(VIII)}$$

worin

$Q^{(+)}$ ein metallisches Kation bedeutet,

umsetzt,

wobei man eine Verbindung der allgemeinen Formel I erhält, in der A $-CH_2-$ oder eine Bindung, B =CH- und $R^1$ H bedeuten,

und/oder daß man

f) eine Verbindung der allgemeinen Formel I mit B = =CH- zu einer Verbindung der allgemeinen Formel I mit B = $-CH_2-$ hydriert

g) und daß man gegebenenfalls eine erhaltene Verbindung der allgemeinen Formel I mit $R^1$ = H verestert zu einer Verbindung der allgemeinen Formel I mit $R^1$ = Alkyl mit 1 - 4 C-Atomen, Aryl mit 6 - 12 C-Atomen oder $-C_6H_4-NH-CO-R^3$ oder eine erhaltene Verbindung der allgemeinen Formel I mit $R^1$ = Alkyl mit 1 - 4 C-Atomen, Aryl mit 6 - 12 C-Atomen oder $-C_6H_4-NH-CO-R^3$ verseift zu einer Verbindung der allgemeinen Formel I mit $R^1$ = H und/oder eine als Racemat erhaltene Verbindung der allgemeinen Formel I in ihre Enantiomeren aufspaltet und/oder eine erhaltene Verbindung der allgemeinen Formel I mit $R^1$ = H durch Umsetzen mit einer Base in eines ihrer physiologisch unbedenklichen Metall- oder Ammoniumsalze überführt.

3. Verfahren zur Herstellung pharmazeutischer Zubereitungen, dadurch gekennzeichnet, daß man mindestens eine Verbindung der allgemeinen Formel I und/oder eines ihrer physiologisch unbedenklichen Metall- oder Ammoniumsalze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

4. Pharmazeutische Zubereitung, gekennzeichnet durch einen Gehalt an mindestens einer Verbindung der allgemeinen Formel I und/oder einem ihrer physiologisch unbedenklichen Metall- oder Ammoniumsalze.

5. Verbindungen der allgemeinen Formel I zur Bekämpfung von Krankheiten.

6. Verwendung von Verbindungen der allgemeinen Formel I bei der Bekämpfung von Krankheiten.

0084775

Nummer der Anmeldung

Europäisches Patentamt

# EUROPÄISCHER RECHERCHENBERICHT

EP 83 10 0045

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | FR-A-2 351 112 (WELLCOME) * Insgesamt * --- | 1-6 | C 07 C 149/26<br>C 07 D 307/935<br>C 07 C 177/00<br>C 07 D 333/18<br>C 07 D 213/32<br>A 61 K 31/19<br>A 61 K 31/215<br>A 61 K 31/557 |
| Y | GB-A-2 012 265 (SANKYO) * Insgesamt * --- | 1-6 | |
| Y | US-A-4 080 458 (H.E. RADUNZ) * Insgesamt * --- | 1-6 | |
| E | EP-A-0 063 779 (CHINOIN) * Insgesamt * --- | 1-6 | |
| P | TETRAHEDRON LETTERS, Band 23, Nr. 20, 1982, Seiten 2135-2138, Pergamon Press Ltd., Oxford, G.B. L. NOVAK et al.: "Synthese of 13-oxa- and 13-thia-PGI2: Metabolically stable and biologically potent PGI2 analogues" * Insgesamt * ----- | 1-6 | RECHERCHIERTE SACHGEBIETE (Int. Cl. 3)<br>C 07 C 149/00<br>C 07 D 307/00<br>C 07 C 177/00 |

Der vorliegende Recherchenbericht wurde für alle Patentansprüche erstellt.

| Recherchenort | Abschlußdatum der Recherche | Prüfer |
|---|---|---|
| DEN HAAG | 19-04-1983 | ALLARD M.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument